(19) Europäisches Patentamt
European Patent Office
Office européen des brevets

(11) EP 0 772 798 B1

# (12) FASCICULE DE BREVET EUROPEEN

(45) Date de publication et mention de la délivrance du brevet:
**02.06.1999 Bulletin 1999/22**

(21) Numéro de dépôt: **95927001.8**

(22) Date de dépôt: **31.07.1995**

(51) Int Cl.$^6$: **G03C 1/685**, C07D 498/10

(86) Numéro de dépôt international:
**PCT/FR95/01031**

(87) Numéro de publication internationale:
**WO 96/04590 (15.02.1996 Gazette 1996/08)**

## (54) COMPOSES PHOTOCHROMIQUES DE STRUCTURE SPIRO INDOLINE- 2,3']-BENZOXAZINE] A GROUPEMENT CYANO OU PHENYLSULFONYL EN 6' ET COMPORTANT UN CYCLE BENZENIQUE CONDENSE EN 7', 8' DU NOYAU BENZOXAZINE, ET LEUR UTILISATION DANS LE DOMAINE DE L'OPTIQUE OPHTALMIQUE

FOTOCHROME VERBINDUNGEN MIT EINER SPIRO(INDOLIN-(2,3')-BENZOXAZIN) STRUKTUR, DIE IN POSITION 6' EINE CYANO- ODER BENZYLSULFONYLGRUPPE UND IN 7',8' DES BENZOXAZINS EINEN KONDENZIERTEN BENZOLRING ENTHÄLT, UND IHRE VERWENDUNG IN DER OPHTHALMISCHEN OPTIK

SPIRO INDOLINE- 2,3']-BENZOXAZINE]-TYPE PHOTOCHROMIC COMPOUNDS CONTAINING A 6'-CYANO OR PHENYLSULPHONYL GROUP AND WITH A 7', 8'-CONDENSED BENZENE RING IN THE BENZOXAZINE RING AND USE OF SAME IN OPHTHALMIC OPTICS

(84) Etats contractants désignés:
**AT CH DE DK ES FR GB IT LI NL SE**

(30) Priorité: **29.07.1994 FR 9409449**

(43) Date de publication de la demande:
**14.05.1997 Bulletin 1997/20**

(73) Titulaire: **TRANSITIONS OPTICAL, INC.**
**Pinellas Park, Florida 34660 (US)**

(72) Inventeurs:
- **LAREGINIE, Pierre**
  **F-13008 Marseille (FR)**
- **LOKSHIN, Vladimir**
  **F-13009 Marseille (FR)**
- **SAMAT, André**
  **F-13013 Marseille (FR)**
- **GUGLIELMETTI, Robert**
  **Boulevard des Alisiers F-13009 Marseille (FR)**
- **ZABALLOS GARCIA, Elena**
  **E-46520 Puerto de Sagunto (ES)**

(74) Mandataire: **Casalonga, Axel et al**
**BUREAU D.A. CASALONGA - JOSSE**
**Morassistrasse 8**
**80469 München (DE)**

(56) Documents cités:
**EP-A- 0 245 020**
**EP-A- 0 316 980**
**JP-A- 3 251 587**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 772 798 B1

## Description

L'invention a pour objet de nouveaux composés photochromiques du type spiro[indoline-[2,3']-benzoxazine] à groupement cyano ou phénylsulfonyl en 6' comportant un cycle benzénique condensé en 7', 8' du noyau benzoxazine, et leur utilisation dans le domaine de l'optique ophtalmique, en particulier dans et/ou sur les lentilles ophtalmiques.

Le photochromisme est un phénomène connu depuis de nombreuses années. On dit qu'un composé est photochromique lorsque ce composé, irradié par un faisceau lumineux, dont certaines longueurs d'onde se situent dans le domaine de l'ultraviolet, change de couleur et revient à sa couleur originelle dès que l'irradiation cesse.

Les applications de ce phénomène sont multiples, mais une des applications connues plus particulièrement intéressante concerne le domaine de l'optique ophtalmique.

De tels composés sont utilisables dans la fabrication de lentilles ou verres pour lunettes en vue de filtrer les radiations lumineuses en fonction de leur intensité.

L'incorporation des composés photochromiques dans un matériau organique constituant une lentille ophtalmique, permet d'obtenir un verre dont le poids est considérablement réduit par rapport aux lentilles classiques en verre minéral qui comportent des halogénures d'argent à titre d'agent photochrome. Leur incorporation dans des matériaux organiques a toujours posé des difficultés techniques.

Toutefois, tout composé à propriété photochromique n'est pas forcément utilisable dans le domaine de l'optique ophtalmique. En effet, le composé photochromique doit répondre à un certain nombre de critères, dont entre autres :

- une forte colorabilité qui est la mesure de la capacité pour un composé photochromique de présenter une couleur intense après isomérisation;
- une coloration après absorption de la lumière rendant apte le composé photochromique, seul ou en combinaison avec d'autres composés photochromiques à être utilisé dans des verres ou lentilles ophtalmiques;
- une absence de coloration ou très faible coloration sous la forme initiale;
- une cinétique rapide de coloration ou de décoloration;
- un photochromisme se manifestant dans une plage de température la plus large possible, et en particulier de préférence entre 0 et 40°C.

Les composés photochromiques organiques connus et utilisés actuellement présentent généralement un photochromisme décroissant lorsque la température augmente, de sorte que le photochromisme est particulièrement marqué à des températures proches de 0°C, alors qu'il est beaucoup plus faible, voire inexistant, à des températures de l'ordre de 40°C qui sont des températures que peuvent atteindre les verres lors notamment de l'exposition au soleil.

Un autre problème rencontré pour les composés photochromiques de l'état de la technique est leur durée de vie. On constate en effet pour certains produits de l'état de la technique, une durée de vie relativement réduite. En effet, après un certain nombre de cycles de coloration et de décoloration, le composé ne présente plus les propriétés photochromes réversibles.

Les propriétés photochromiques des spirooxazines ont été décrites par R.E.FOX dans le document Final Report of Contact AF 41, A.D. 440226 1961. 657.

Des composés du type spiro(indoline-naphtoxazine) ont été synthétisés et décrits notamment dans l'article de N.Y.C. CHU "Photochromism : Molecules and Systems" Ed. H. Dūrr, H. Bovas Laurent, Elsevier, Amsterdam 1990, ch. 24, ainsi que des composés du type spiro(indoline-quinazolinoxazine) ou spiro(indoline-benzo-thiazolooxazine) dans les brevets US 5139707 et US 5114621 (R. Guglielmetti, P. Tardieu) délivrés au nom de la Société ESSILOR.

Des composés du type spiro[indoline-[2,3']-benzoxazinc] photochromiques ont également été synthétisés et décrits dans la demande de brevet EP-0245020.

La demande de brevet japonais JP 3251587 divulgue par ailleurs des composés photochromiques du type indolinospiropyridobenzoxazine porteurs, en position 6' du noyau benzoxazine, de groupements CN, $CF_3$ ou alkoxycarbonyle, et dont l'azote indolinique est substitué par des groupements alkoxycarbonyle alkyle.

Ces composés sont phtochromiques à des températures de l'ordre de 30 à 40°C.

Ces composés, sous leur forme ouverte, présentent un spectre d'absorption dans le visible comportant un maximum d'absorption à une longueur d'onde λmax variant de 600 à 617 nm.

Il est souhaitable d'obtenir des composés dont le spectre d'absorption dans le visible, pour leur forme ouverte, comporte un maximum d'absorption à des longueur d'onde plus élevées que les composés de la demande de brevet JP 3251587.

En effet, le déplacement du λmax vers des valeurs plus élevées conduit à des composés photochromiques de couleur approchant le vert, sous leur forme ouverte, cette couleur étant recherchée pour une application ophtalmique.

La demanderesse a découvert une nouvelle famille de composés de structure spiro[indoline-[2,3']-benzoxazine] à groupement cyano ou phénylsulfonyl en position 6' et comportant un cycle benzénique condensé en 7', 8' du noyau benzoxazine, présentant des propriétés photochromiques particulièrement intéressantes.

Les composés de l'invention présentent en effet une forte colorabilité, sous leur forme ouverte, dans le domaine du visible. Leurs colorations sont bleu-vert. Ils peuvent ainsi être utilisés avec d'autres composés photochromiques donnant une couleur rouge en vue d'obtenir une coloration naturelle lors de l'exposition à la lumière.

Les composés conformes à l'invention, sous leur forme colorée, présentent un déplacement vers des longueurs d'ondes plus élevées du spectre d'absorption dans le visible par rapport aux composés homologues de l'art antérieur de structure spiro[indoline-[2,3']-benzoxazine] non substitués en 6' ou de structure spiro[indoline-[2,3']-benzoxazine] substitués en 6' de la demande JP 3251587. Les longueurs d'onde correspondant au maximum d'absorption sont supérieures à 630 nm, et le plus souvent supérieures à 640 nm.

Les composés selon l'invention présentent de fortes bandes d'absorption dans la région des rayonnements UV visibles, sous leur forme fermée, ainsi qu'un déplacement vers des longueurs d'onde plus élevées des bandes d'absorption du spectre UV de la forme fermée par rapport aux homologues spiro[indoline-[2,3']-benzoxazine] non substitués en 6' de l'art antérieur.

La plupart des rayonnements UV étant bloqués par les vitrages classiques à l'exception des rayonnements UV visibles, les composés de l'invention présentent ainsi une bonne colorabilité même si un vitrage est interposé entre la source de rayonnements UV (lumière naturelle) et le composé photochromique.

Les composés conformes à l'invention présentent par ailleurs une absence de coloration ou une très faible coloration à l'état initial et une cinétique rapide de coloration et de décoloration dans une plage de températures large, comprise en particulier entre 0 et 40°C. Leurs propriétés photochromiques sont aussi remarquables à des températures de l'ordre de 20°C qu'à des températures de l'ordre de 35°C. Il en résulte qu'ils sont particulièrement adaptés pour les lentilles de contact dont la température d'utilisation est de l'ordre de 35°C et pour les verres de lunettes lors de l'exposition au soleil.

La demanderesse a également constaté que ces composés avaient une durée de vie particulièrement longue.

Toutes ces propriétés font que ces nouveaux composés photochromiques sont particulièrement intéressants dans leur utilisation dans l'optique ophtalmique et en particulier pour leur utilisation dans et/ou sur des lentilles ophtalmiques.

On appelle lentilles ophtalmiques au sens de l'invention, des verres de lunettes, en particulier des verres solaires, des lentilles de contact.

Un objet de l'invention est donc constitué par les nouveaux composés photochromiques.

Un autre objet de l'invention est constitué par leur utilisation dans l'optique ophtalmique.

L'invention a également pour objet des compositions destinées à être utilisées pour le revêtement de lentilles ophtalmiques ou leur incorporation dans ces lentilles.

D'autres objets de l'invention apparaîtront à la lecture de la description et des exemples qui suivent.

Les composés photochromiques conformes à l'invention sont des composés de structure spiro[indoline-[2,3']-benzoxazine] essentiellement caractérisés par le fait qu'ils sont substitués en position 6' par un groupement $R_6$ qui est un groupement cyano ou un groupement phénylsulfonyl lié par l'atome de soufre au carbone 6, et possèdent un cycle benzénique condensé en position 7', 8' du noyau benzoxazine.

On entend dans la présente invention par groupement phénylsulfonyl, le radical phénylsulfonyl, et tout dérivé de celui-ci, et en particulier les radicaux phénylsulfonyl substitués.

Les composés photochromiques conformes à l'invention sont choisis de préférence parmi ceux répondant à la formule (I) suivante :

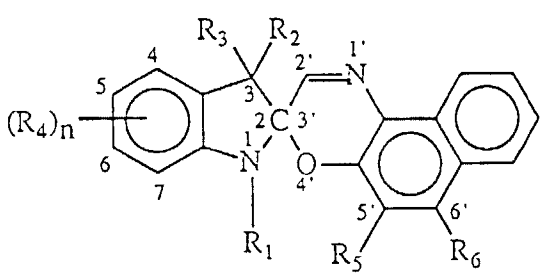

(I)

dans laquelle :

n varie de 0 à 4;

$R_1$ représente :

i) un groupe alkyle de 1 à 16 atomes de carbone tel qu'un groupe méthyle, éthyle, n-propyle, isopropyle, n-butyle;
ii) un groupe allyle, phényle, arylalkyle tel qu'un groupe benzyle, phényle mono- ou disubstitué par des substituants du type alkyle ou alcoxy de 1 à 6 atomes de carbone ou des atomes d'halogène tels que chlore;
iii) un groupe alicyclique tel qu'un groupe cyclohexyle, éventuellement substitué;
iv) un groupe hydrocarbure aliphatique comportant dans sa chaîne un ou plusieurs hétéroatomes tels que O, N ou S, notamment une fonction acide, ester ou alcool;

$R_2$ et $R_3$ peuvent chacun et indépendamment l'un de l'autre représenter un groupe $C_1$-$C_8$ alkyle, phényle, phényle mono- ou disubstitué par des groupes $C_1$-$C_4$ alkyle et/ou $C_1$-$C_5$ alcoxy, ou peuvent être combinés pour former une chaîne cyclique de 6 à 8 atomes de carbone;
$R_4$ et $R_5$ représentent, indépendamment l'un de l'autre :

i) un atome d'hydrogène, une fonction amine NR'R", où R' et R" représentent chacun et indépendamment un atome d'hydrogène, un groupe alkyle, cycloalkyle, phényle ou un dérivé substitué de celui-ci; R' et R" peuvent se combiner pour former un cycloalkyle pouvant être substitué et contenir un ou plusieurs hétéroatomes;
ii) un groupe R, OR, SR, COR ou COOR, dans lequel R représente un atome d'hydrogène, un groupe alkyle de 1 à 6 atomes de carbone ou un groupe aryle ou hétéroaryle;
iii) un atome d'halogène, un groupe $C_1$-$C_4$ monohaloalkyle, l'halogène étant notamment Cl ou Br, ou un groupe $C_1$-$C_4$ polyhaloalkyle tel que $CF_3$;
iv) $-NO_2$, CN, SCN;
v) un groupement

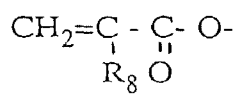

avec $R_8$ représentant H ou $CH_3$;
vi) un groupement

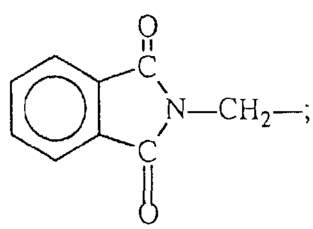

chacun des substituants $R_4$ pouvant être présent sur l'un quelconque des atomes de carbone convenables de la partie indoline du composé photochromique, en positions 4, 5, 6 et 7, quand l'autre est un atome d'hydrogène, alors que quand n=2, il est préférable que les substituants soient présents en positions 4 et 5, 5 et 6, 4 et 6 ou 6 et 7 ;
$R_6$ est choisi parmi les groupements CN,

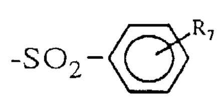

formule dans laquelle $R_7$ désigne un atome d'hydrogène, un alkyle ayant de 1 à 6 atomes de carbone, un alcoxy ayant de 1 à 6 atomes de carbone, ou un halogène.

De préférence $R_7$ est en position para du groupement $SO_2$. $R_7$ est préférentiellement un atome d'hydrogène.

Les composés préférés répondent à la formule suivante :

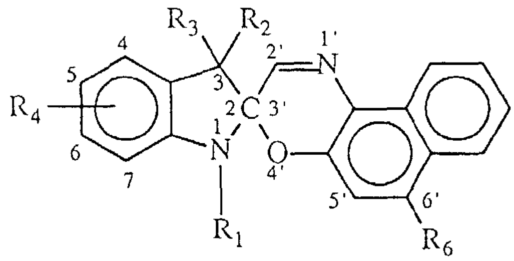

(I')

dans laquelle :

$R_1$ désigne un groupement alkyle ayant de 1 à 16 atomes de carbone ou un groupement allyle;
$R_2$ et $R_3$ représentent chacun et indépendamment l'un de l'autre un groupement alkyle ayant de 1 à 8 atomes de carbone;
$R_4$ désigne un atome d'halogène, un atome d'hydrogène ou un radical alcoxy ayant de 1 à 6 atomes de carbone, un groupement

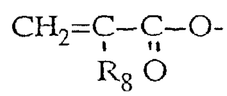

avec $R_8$ désignant H ou $CH_3$,
ou un groupement

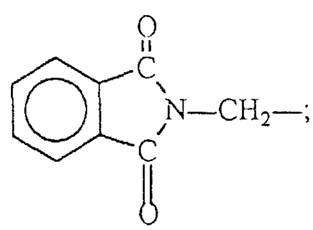

$R_6$ a la signification mentionnée précédemment dans la formule (I).
Dans les composés de l'invention, $R_6$ est de préférence un groupement cyano. $R_4$ est de préférence en position 5 du groupement indoline et désigne un groupement alcoxy, halogène, ou un groupement

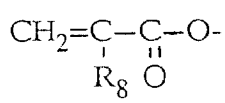

avec $R_8$ désignant H ou $CH_3$,
et/ou l'un au moins des groupements $R_1$, $R_2$ est un groupement alkyle comportant au moins 2 atomes de carbone.

De tels composés présentent des colorations intenses lorsqu'ils sont introduits en matrice.

Les composés plus particulièrement préférés répondent à la formule suivante :

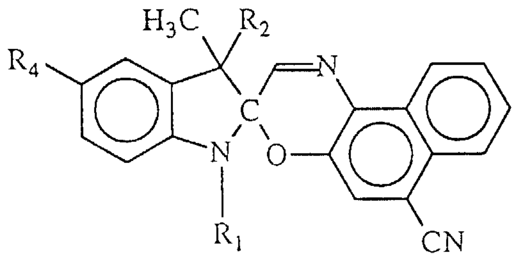

(I")

dans laquelle :

$R_1$ désigne un alkyle ayant de 1 à 16 atomes de carbone ou un groupe allyle;
$R_2$ désigne un alkyle ayant de 1 à 8 atomes de carbone;
$R_4$ désigne un atome d'halogène, un atome d'hydrogène, un radical alcoxy ayant de 1 à 6 atomes de carbone, ou un radical

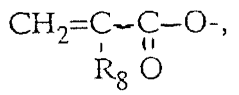

avec $R_8$ désignant H ou $CH_3$.
Dans la formule (I") définie ci-dessus :

$R_1$ désigne préférentiellement un radical méthyle, hexadécyle ou allyle;
$R_2$ désigne de préférence un radical méthyle ou éthyle;
$R_4$ désigne préférentiellement l'hydrogène, le chlore ou le radical méthoxy, ou le radical

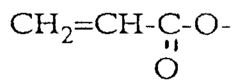

Les composés selon l'invention peuvent être obtenus selon un procédé comprenant la condensation d'une base de Fischer avec un orthoaminophénol substitué en position 4 par un groupement cyano ou benzylsulfonyl dans un milieu solvant tel que le toluène en présence d'un agent oxydant tel que le diméthylsulfoxyde

En particulier les composés de formule (I) selon l'invention peuvent être obtenus selon ce procédé, par condensation d'une base de Fischer de formule :

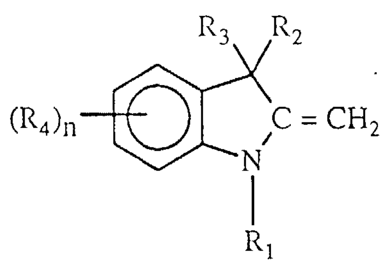

(II)

dans laquelle $R_1$, $R_2$, $R_3$, $R_4$ et n ont les mêmes significations indiquées ci-dessus, avec un orthoaminophénol annélé de formule :

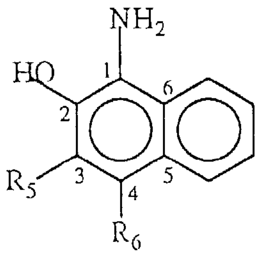

(III)

où $R_5$, $R_6$ ont les mêmes significations indiquées ci-dessus, dans un milieu solvant tel que le toluène, en présence d'un agent oxydant tel que le diméthylsulfoxyde.

Selon ce procédé, on utilise de préférence également un agent déshydratant tel que le sulfate de calcium ou de magnésium et/ou un agent d'activation de l'agent oxydant tel que l'hydrogénocarbonate de sodium, de manière à favoriser la réaction de condensation et/ou à augmenter le rendement en produit final.

La température de réaction est comprise de préférence entre 50 et 80°C environ.

Ce procédé peut être représenté par le schéma réactionnel suivant :

SCHEMA REACTIONNEL

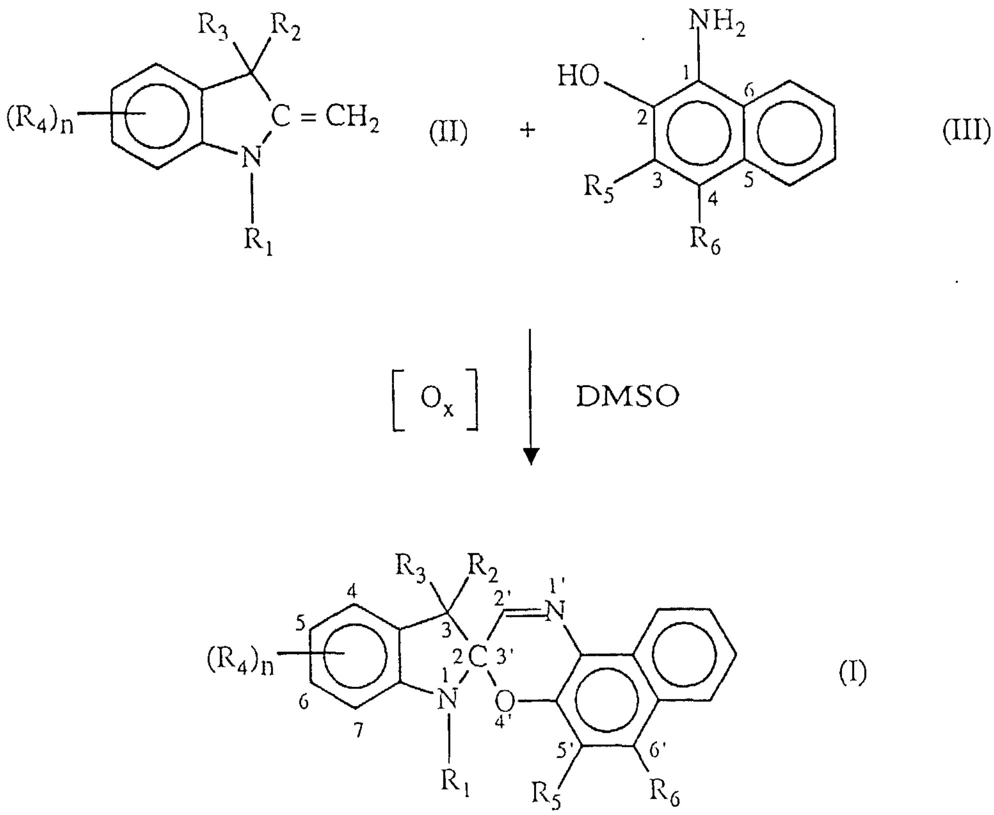


(II) + (III)

(I)

Les composés orthoaminophenol de formule (III), substitués en position 4 par un groupement cyano peuvent être obtenus en faisant réagir un composé de formule :

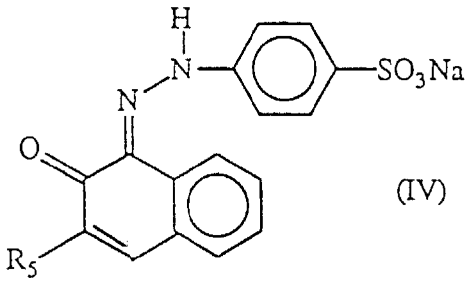

(IV)

où $R_5$ a la même signification indiquée ci-dessus, avec un cyanure de métal alcalin tel que sodium ou potassium, pendant quelques jours à 90°C, puis on filtre et on sature de dioxyde de carbone.

Les composés ortho aminophénol de formule (III), dans lesquels $R_6$ est un groupement phénylsulfonyl peuvent être obtenus en procédant selon le schéma suivant :

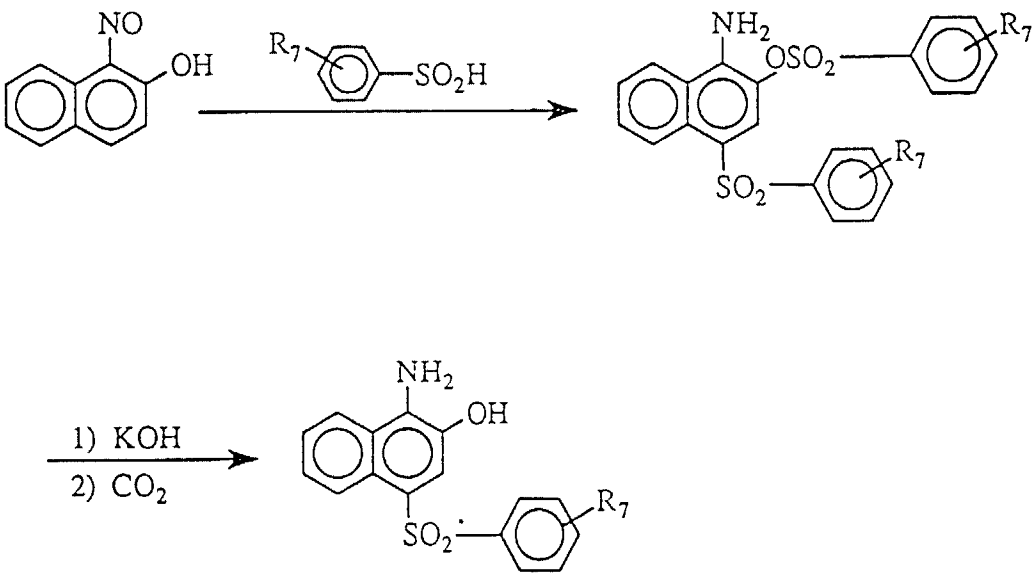


Les composés photochromiques de l'invention peuvent être utilisés pour réaliser des lentilles ophtalmiques photochromiques.

Les composés conformes à l'invention peuvent être introduits dans une composition destinée à être appliquée sur ou être introduite dans un matériau polymère organique transparent pour obtenir un article transparent photochromique. Ils peuvent également être introduits dans des compositions solides telles que films plastique, plaques et lentilles pour réaliser des matériaux utilisables, notamment comme lentilles ophtalmiques, lunettes de soleil, viseurs, optique de caméra et filtres.

Les compositions liquides qui constituent un objet de l'invention sont essentiellement caractérisées par le fait qu'elles contiennent sous forme dissoute ou dispersée les composés conformes à l'invention dans un milieu à base de solvants appropriés pour être appliqués ou introduits dans un matériau polymère transparent.

Des solvants plus particulièrement utilisables sont des solvants organiques choisis parmi le benzène, le toluène, le chloroforme, l'acétate d'éthyle, la méthyléthylcétone, l'acétone, l'alcool éthylique, l'alcool méthylique, l'acétonitrile, le tétrahydrofurane, le dioxane, l'éther méthylique d'éthylèneglycol, le diméthylformamide, le diméthylsulfoxyde, le méthylcellosolve, la morpholine et l'éthylène glycol.

Lorsque les composés conformes à l'invention sont dispersés, le milieu peut également contenir de l'eau.

Selon une autre forme de réalisation, les composés conformes à l'invention peuvent être introduits et de préférence dissous dans des solutions incolores ou transparentes préparées à partir de polymères, de copolymères ou de mélanges de polymères transparents dans un solvant organique approprié.

Les exemples de telles solutions sont entre autres des solutions de nitrocellulose dans l'acétonitrile, de polyvinylacétate dans l'acétone, de chlorure de polyvinyle dans la méthyléthylcétone, de polyméthylformamide, de polyvinylpyrrolidone dans de l'acétonitrile, de polystyrène dans le benzène, d'éthylcellulose dans du chlorure de méthylène.

Ces compositions peuvent être appliquées sur des supports transparents tels qu'en téréphtalate de polyéthylèneglycol, de papier borylé, de triacétate de cellulose et séchées pour obtenir un matériau photochromique qui peut se colorer en présence d'une radiation ultraviolette, et qui retourne à l'état non coloré et transparent en l'absence de la source de radiation.

Les composés photochromiques de la présente invention ou les compositions les contenant définies ci-dessus, peuvent être appliqués ou incorporés dans un matériau organique polymérisé transparent solide approprié pour des éléments ophtalmiques tels que des lentilles ophtalmiques ou des matériaux utiles pour être utilisés dans des lunettes de soleil, des viseurs, des optiques de caméras et des filtres.

A titre de matériaux solides transparents, qui peuvent être utilisés pour réaliser des lentilles ophtalmiques conformes à l'invention, on peut citer les polymères de polyol(allylcarbonate), des polyacrylates, des poly(alkylacrylate) tels que des polyméthylméthacrylates, l'acétate de cellulose, le triacétate de cellulose, le propionate acétate de cellulose, le butyrate acétate de cellulose, le poly(vinylacétate), le poly(vinylalcool), les polyuréthanes, les polycarbonates, les polyéthylènetéréphtalates, les polystyrènes, les (polystyrèneméthylméthacrylate), les copolymères de styrène et d'acrylonitrile, les polyvinylbutyrates.

Les copolymères transparents ou des mélanges de polymères transparents sont également appropriés pour réaliser de tels matériaux.

On peut citer à ce sujet les matériaux préparés à partir de polycarbonates tels que le poly(4,4'-dioxyphénol-2,2 propane), le polyméthylméthacrylate, les polyol(allylcarbonate) tels qu'en particulier le diéthylèneglycol bis(allylcarbonate) et ses copolymères tels que par exemple avec l'acétate de vinyle. On peut citer en particulier les copolymères de diéthylèneglycol bis(allylcarbonate) et d'acétate de vinyle (80-90/10-20) et encore le copolymère de diéthylèneglycol bis(allylcarbonate) avec l'acétate de vinyle, l'acétate de cellulose et le propionate de cellulose, le butyrate de cellulose (80-85/15-20).

Les polyols(allylcarbonate) sont préparés en utilisant les allylcarbonates de polyols liquides aliphatiques ou aromatiques, linéaires ou ramifiés tels que les glycols aliphatiques de bis-allylcarbonate ou les alkylène bis(allylcarbonate). Parmi les polyols (allylcarbonate) qui peuvent être utilisés pour préparer les matériaux transparents solides utilisables conformément à l'invention, on peut citer l'éthylèneglycol bis(allylcarbonate), le diéthylèneglycol bis(2-méthallylcarbonate), le diéthylèneglycol bis(allylcarbonate), l'éthylèneglycol bis(2-chloroallylcarbonate), le triéthylèneglycol bis(allylcarbonate), le 1,3-propanediol bis(allylcarbonate), le propylèneglycol bis(2-éthylallylcarbonate), le 1,3-butanediol bis(allylcarbonate), le 1,4-butanediol bis(2-bromoallylcarbonate), le dipropylèneglycol bis(allylcarbonate), le triméthylèneglycol bis(2-éthylallylcarbonate), le pentaméthylèneglycol bis(allylcarbonate), l'isopropylène bisphénol bis (allylcarbonate). Le produit le plus important est constitué par le dièthylèneglycol bis(allylcarbonate) encore connu sous la dénomination CR39.

La quantité de composés photochromiques à utiliser conformément à l'invention, soit dans la composition, soit au moment de son introduction dans le support solide, n'est pas critique et dépend généralement de l'intensité de la couleur que la composition peut conférer au matériau après exposition aux radiations. D'une manière générale, plus on ajoute de composés photochromiques, plus la coloration sous irradiation sera importante.

Conformément à l'invention, on utilise une quantité suffisante pour conférer au matériau traité la propriété de changer de couleur au moment de l'exposition à la radiation. Cette quantité de composés photochromiques est généralement comprise entre 0,001 et 20% en poids et de préférence entre 0,05 et 10% en poids par rapport au poids total du matériau optique ou de la composition.

Les composés photochromiques conformes à l'invention peuvent également être introduits dans un support temporaire (tel qu'un vernis formant un revêtement sur un substrat) de transfert et être transférés ensuite thermiquement dans le substrat comme décrit en particulier dans le brevet US-4.286.957 ou US-4.880.667.

Ces composés peuvent être utilisés avec d'autres composés photochromiques, tels que des composés photochromiques donnant lieu à des colorations différentes telles que rouge, connus dans l'état de la technique.

Une fois appliqués sur des matériaux ophtalmiques ou introduits dans de tels matériaux, on constate après exposition aux irradiations UV, l'apparition d'une coloration et le retour à la couleur ou à la transparence originelle lorsqu'on interrompt l'exposition aux radiations UV.

Les composés conformes à l'invention présentent l'intérêt de permettre ce changement de coloration un grand nombre de fois et ceci à des températures très variables comprises entre 0 et 40°C.

Les exemples suivants sont destinés à illustrer l'invention sans pour autant présenter un caractère limitatif.

## EXEMPLES

EXEMPLE 1

6'-cyano-1,3,3-triméthyl-spiro-[indoline-[2,3']-[3H]-napht-[2,1-b]-[1,4]-oxazine] :

**A - Synthèse de la 4-cyano-2-hydroxynaphtylamine :**

Cette synthèse est effectuée selon le schéma réactionnel :

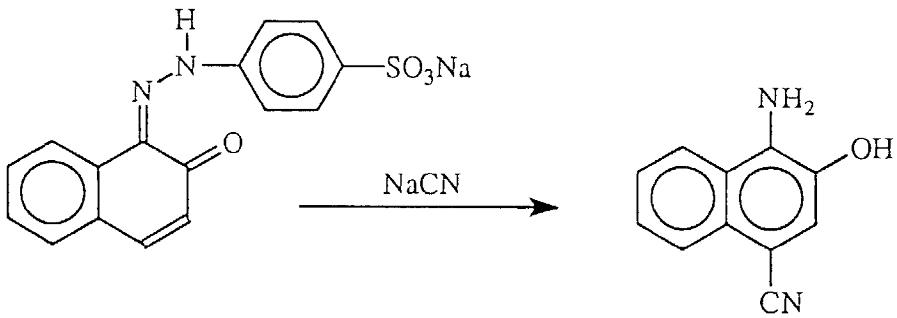


(V)

A une solution du sel de sodium du 4-(2-hydroxy-1-naphtylazo) benzène acide sulfonique (10 g, 28,5 mmoles) dans 120 ml d'eau, on ajoute 30 g (0,46 moles) de cyanure de potassium. Après 2 jours à 90°C, on filtre et sature le filtrat avec du dioxyde de carbone. Le précipité jaune est filtré pour donner 2,9 g (55%) de 4-cyano-2-hydroxynaphtylamine (V).

Point de fusion : 206°C.

**B - Synthèse de la spironaphtoxazine correspondante :**

A un mélange de 2-méthylène-1,3,3-triméthylindoline (0,35 g, 2 mmoles), sulfate de calcium anhydre (0,4 g), diméthylsulfoxyde (0,234g, 3 mmoles), hydrogénocarbonate de sodium (0,5 g), 4-cyano-2-hydroxynaphtylamine (V) (0,405 g, 2,2 mmoles), on ajoute 10 ml de toluène et laisse 12 heures sous agitation à 80°C. On filtre, lave avec du toluène chaud (5 ml) et évapore le filtrat. Le produit est ensuite purifié par chromatographie flash (90 hexane/10 acétate d'éthyle).

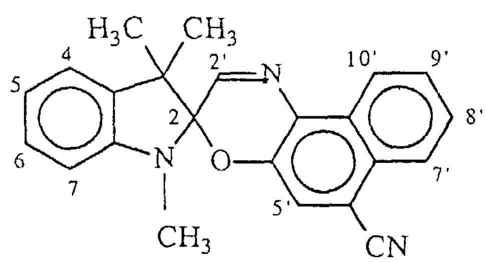

Rendement: 58%

Point de fusion : 159°C.

RMN $^1H$ (250 MHz, $CDCl_3$, TMS), δ ppm : 1,34 (3H, s, 3-$CH_3$); 1,37 (3H, s, 3-$CH_3$); 2,76 (3H, s, N-$CH_3$); 6,59 (1H, d, 7-H); 6,93 (1H, dd, 5-H); 7,09 (1H, d, 4-H); 7,24 (1H, dd, 6-H); 7,46 (1H, s, 5'-H); 7,59 (1H, dd, 8'-H); 7,69 (1H, dd, 9'-H); 7,89 (1H, s, 2'-H); 8,14 (1H, d, 7'-H); 8,66 (1H, d, 10'-H).

RMN $^{13}C$ (62,5 MHz, $CDCl_3$, TMS), δ ppm : 20,9 (q, 3-$CH_3$); 25,6 (q, 3-$CH_3$); 29,9 (q, N-$CH_3$); 52,2 (s, 3-C); 99,0 (s, 2-C); 107,5 (d, 7-C); 120,5 (d, 5-C); 121,7 (d, 4-C); 122,7 (d, 10'-C); 123,4 (d, 5'-C); 125,3 (d, 7'-C); 126,8 (d, 8'-C); 128,4 (d, 6-C); 128,7 (d, 9'-C); 154,6 (d, 2'-C).

FORME OUVERTE (toluène) : (épaulement : 597 nm); $\lambda_{max}$ : 644 nm.

FORME FERMEE (acétonitrile) : $\lambda_{max}$ nm (ε);206 (46700); 231(e) (43700); 236 (46300); 244 (51800); 294 (5000); 317 (5800); 330 (7400); 368 (7200); 386 (5400).

ε représente le coefficient d'extinction molaire de formule générale :

$$\varepsilon = (\log Io/I)\ C.l$$

où

Io est l'intensité lumineuse incidente
I est l'intensité lumineuse transmise
C est la concentration molaire
l est la longueur (cm) de la solution traversée.

EXEMPLE 2

6'-cyano-5-méthoxy-1,3,3-triméthyl-spiro-[indoline-[2,3']-[3H]-napht-[2,1-b]-[1,4]-oxazine] :

A un mélange de 5-méthoxy-2-mèthylène-1,3 3-triméthylindoline (0,203 g 1 mmole), chlorhydrate de 4-cyano-2-hydroxynaphtylamine (0,255 g, 1,1 mmole), triéthylamine (0,152 g, 1,5 mmole), sulfate de calcium anhydre (0,2 g), diméthylsulfoxyde (0,117 g, 1,5 mmole), hydrogénocarbonate de sodium (0,25 g), on ajoute 5 ml de toluène et laisse 12 heures sous agitation à 80°C. On filtre, lave avec du toluène chaud (2 ml), et évapore le filtrat. Le produit est ensuite purifié par chromatographie flash (90 hexane/10 acétate d'éthyle).

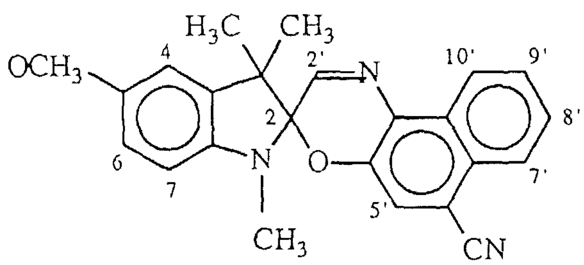

Rendement : 65%
Point de fusion : 128°C.

RMN $^{1}H$ (250 MHz, $CDCl_3$, TMS), δ ppm : 1,33 (3H, s, 3-$CH_3$); 1,38 (3H, s, 3-$CH_3$); 2,69 (3H, s, N-$CH_3$); 3,81 (3H, s, O-$CH_3$); 3,50 (1H, d, 7-H); 6,72-6,78 (2H, m, 4 et 6-H); 7,47 (1H, s, 5'-H); 7,59 (1H, dd, 8'-H); 7,70 (1H, dd, 9'-H); 7,88 (1H, s, 2'-H); 8,13 (1H, d, 7'-H); 8,66 (1H, d, 10'-H).

RMN $^{13}C$ (62,5 MHz, $CDCl_3$, TMS), δ ppm : 20,7 (q, 3-$CH_3$); 25,5 (q, 3-$CH_3$); 30,1 (q, N-$CH_3$); 52,4 (s, 3-C); 56,0 (q, O-$CH_3$); 99,4 (s, 2-C); 107,7 (d, 7-C); 109,4 (d, 4 ou 6-C); 112,0 (d, 4 ou 6-C); 122,5 (d, 10'-C); 123,3 (d, 5'-C); 125,2 (d, 7'-C); 126,7 (d, 8'-C); 128,5 (d, 9'-C); 154,3 (d, 2'-C).

FORME OUVERTE (épaulement : 606 nm); $\lambda_{max}$ (toluène) : 659 nm.

FORME FERMEE (acétonitrile) : $\lambda_{max}$ nm (ε):203 (62600); 231(e) (47600); 236 (51600); 244 (56600); 316 (8800); 329 (8200); 368 (7500); 371 (7500); 388(e) (5300).

EXEMPLE 3

5-chloro-6'-cyano-1,3,3-triméthyl-spiro-[indoline-[2,3']-[3H]-napht-[2,1-b]-[1,4]-oxazine] :

Obtenue par réaction de la 4-cyano-2-hydroxynaphtylamine (V) sur la 5-chloro-2-méthylène-1,3,3-triméthylindoline.

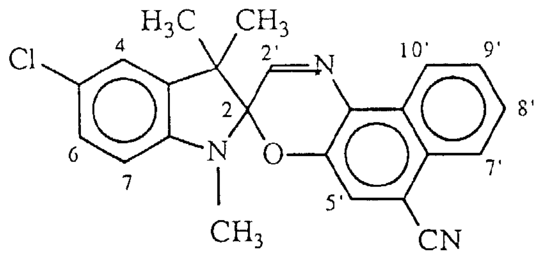

Rendement : 50%

Point de fusion : 175°C.

RMN $^1H$ (250 MHz, $CDCl_3$, TMS), δ ppm : 1,32 (3H, s, 3-$CH_3$); 1,37 (3H, s, 3-$CH_3$); 2,73 (3H, s, N-$CH_3$); 6,49 (1H, d, 7-H); 7,04 (1H, d, 4-H); 7,18 (1H, dd, 6-H); 7,46 (1H, s, 5'-H); 7,60 (1H, dd, 8'-H); 7,70 (1H, dd, 9'-H); 7,87 (1H, s, 2'-H); 8,14 (1H, d, 7'-H); 8,65 (1H, d, 10'-H). RMN $^{13}C$ (62,5 MHz, $CDCl_3$, TMS), δ ppm : 20,6 (q, 3-$CH_3$); 25,2 (q, 3-$CH_3$); 29,7 (q, N-$CH_3$); 52,2 (s, 3-C); 98,9 (s, 2-C); 108,2 (d, 7-C); 122,1 (d, 4-C); 122,5 (d, 10'-C); 123,0 (d, 5'-C); 125,1 (d, 7'-C); 126,7 (d, 8'-C); 127,9 (d, 6-C); 128,5 (d, 9'-C); 153,7 (d, 2'-C).

FORME OUVERTE : $\lambda_{max}$ (toluène) : 644 nm.

FORME FERMEE (acétonitrile) : $\lambda_{max}$ nm (ε):206 (47500); 231 (47400); 235 (47300); 244 (48500); 316 (7500); 330 (8000); 367 (7900); 370(e) (7700); 384(e) (5900).

EXEMPLE 4

6'-cyano-1,3-diméthyl-3-éthyl-spiro-[indoline-[2,3']-[3H]-napht-[2,1-b]-[1,4]oxazine] :

Obtenue par réaction de la 4-cyano-2-hydroxynaphtylamine (V) sur la 1,3-diméthyl-3-éthyl-2-méthylèneindoline.

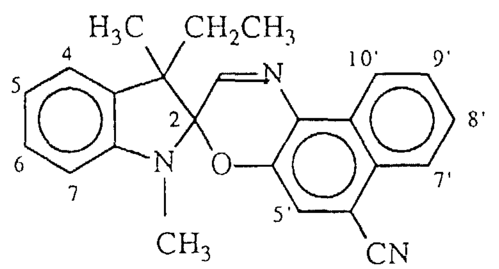

Rendement : 42%

Point de fusion : 162°C.

RMN $^1H$ (250 MHz, $CDCl_3$, TMS), δ ppm (δ ppm du 2° diastéréoisomère) : 0,83 (0,87) (3H, t, 3-$CH_2CH_3$); 1,40 (1,29) (3H, s, 3-$CH_3$); 1,63-2,17 (2H, m, 3-$CH_2$); 2,61 (2,75) (3H, s, N-$CH_3$); 6,53 (6,60) (1H, d, 7-H); 6,92 (6,91) (1H, dd, 5-H); 7,10 (7,08) (1H, d, 4-H); 7,22 (1H, dd, 6-H); 7,48 (7,46) (1H, s, 5'-H); 7,53-7,65 (2H, m, 8' et 9'-H); 7,91 (7,90) (1H, s, 2'-H); 8,14 (8,12) (1H, d, 7'-H); 8,69 (8,66) (1H, d, 10'-H).

FORME OUVERTE : $\lambda_{max}$(toluène) : 643 nm.

FORME FERMEE (acétonitrile) : $\lambda_{max}$ nm (ε) : 206 (47100); 230(e) (45000); 236 (48500); 244 (54200); 294 (5600); 317(e) (6400); 330 (8000); 367 (8100); 371(e) (8000); 386(e) (6200).

EXEMPLE 5

1-allyl-6'-cyano-3,3-diméthyl-spiro-[indoline-[2,3']-[3H]-napht-[2,1-b]-[1,4]-oxazine] :

Obtenue par réaction de la 4-cyano-2-hydroxynaphtylamine (V) sur la 1-allyl-3,3-diméthyl-2-méthylèneindo-

line.

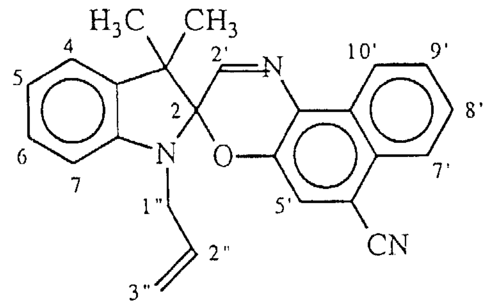

Rendement : 34%

Point de fusion : 149°C.

RMN $^{1}H$ (250 MHz, $CDCl_3$, TMS), δ ppm : 1,33 (3H, s, 3-$CH_3$); 1,39 (3H, s, 3-$CH_3$); 3,70-3,95 (2H, m, 1"-H); 5,05-5,30 (2H, m, 3"-H); 5,75-5,95 (1H, m, 2"-H); 6,62 (1H, d, 7-H); 6,92 (1H, dd, 5-H); 7,09 (1H, dd, 4-H); 7,20 (1H, dd, 6-H); 7,43 (1H, s, 5'-H); 7,58 (1H, dd, 8'-H); 7,68 (1H, dd, 9'-H); 7,88 (1H, s, 2'-H); 8,13 (1H, d, 7'-H); 8,64 (1H, d, 10'-H).

RMN $^{13}C$ (62,5 MHz, $CDCl_3$, TMS), δ ppm : 20,8 (q, 3-$CH_3$); 25,6 (q, 3-$CH_3$); 46,9 (t, 1"); 52,7 (s, 3-C); 99,3 (s, 2-C); 107,9 (d, 7-C); 116,7 (t, 3"-C); 120,2 (d, 5-C); 121,5 (d, 4-C); 122,5 (d, 10'-C); 123,2 (d, 5'-C); 125,1 (d, 7'-C); 126,6 (d, 8'-C); 128,1 (d, 6-C); 128,4 (d, 9'-C); 133,9 (d, 2'-C).

FORME OUVERTE : $\lambda_{max}$ (toluène) : 645 nm.

FORME FERMEE (acétonitrile) : $\lambda_{max}$ (ε) : 205 (46000); 236 (48000); 245 (53300); 318 (5900); 331 (7500); 368 (7700); 371 (7700); 385(e) (6200).

EXEMPLE 6

6'-cyano-3,3-diméthyl-1-hexadécyle-spiro-[indoline-[2,3']-[3H]-napht-[2,1-b]-[1,4]-oxazine] :

Obtenue par réaction de la 4-cyano-2-hydroxynaphtylamine sur la 3,3-diméthyl-1-hexadécyl-2-méthylèneindoline.

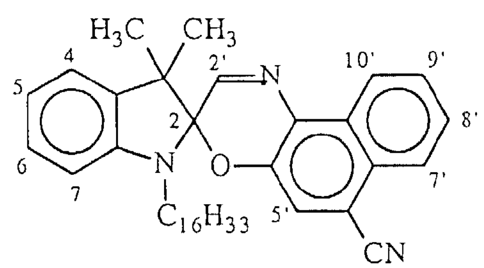

Huile

Rendement : 54%.

RMN $^{1}H$ (250 MHz, $CDCl_3$, TMS), δ ppm : 1,08-1,12 (m, $C_{15}H_{31}$); 1,19 (3H, s, 3-$CH_3$); 1,23 (3H, s, 3-$CH_3$); 3,02 (2H, t, N-$CH_2$); 6,58 (1H, d, 7-H); 6,95 (1H, dd, 5-H); 7,09 (1H, d, 4-H); 7,25 (1H, ddd, 6-H); 7,47 (1H, s, 5'-H); 7,59 (1H, dd, 8'-H); 7,70 (1H, dd, 9'-H); 7,88 (1H, s, 2'-H); 8,14 (1H, d, 7'-H); 8,68 (1H, d, 10'-H).

FORME OUVERTE : $\lambda_{max}$ (toluène) : 647 nm.

EXEMPLE 7 (comparatif)

Composé 1,3,3-triméthyl-spiro-[indoline-[2,3']-[3H]-napht-[2,1-b]-[1,4]-oxazine]

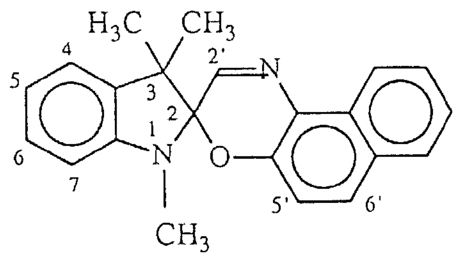

**I.PROPRIETES PHOTOCHROMIQUES EN SOLUTION DES COMPOSES DES EXEMPLES 1 à 7**

PARAMETRES SPECTROCINETIQUES (mesurés dans le toluène à 25°C à une concentration de 2,5 x $10^{-5}$ M)

| EXEMPLES | $\lambda_{max}$(nm) | Constante cinétique de décoloration thermique $k_{\Delta}$ en $s^{-1}$ | $A_o$ Colorabilité |
|---|---|---|---|
| 1 | 644 | 0,22 | 1,03 |
| 2 | 659 | 0,20 | 0,9 |
| 3 | 644 | 0,32 | 1,05 |
| 4 | 643 | 0,14 | 0,86 |
| 5 | 645 | 0,33 | 1,06 |
| 6 | 647 | 0,33 | |
| 7 (comparatif) | 590 | | |

Les paramètres spectrocinétiques sont déterminés comme suit.

On irradie par le rayonnement UV des solutions toluéniques contenant chacune l'un des composés des exemples 1 à 7.

Les mesures sont effectuées à 25°C (± 0,2°C contrôlée par un thermostat extérieur du type Hubert-ministat) dans une cuve en quartz cylindrique de section 10 mm et de longueur optique 10 cm.

L'enregistrement du spectre d'absorption dans le visible à l'aide d'un spectromètre rapide du type WARNER-SWASEY permet de déterminer $\lambda_{max}$ (longueur d'onde au maximum d'absorption) et Ao (absorbance initiale ou densité optique au $\lambda_{max}$) mesurées tout de suite après le flash de coloration. La diminution de Ao en fonction du temps permet de calculer la constante de cinétique de décoloration thermique $k_{\Delta}$.

Pour cela on effectue une photolyse des échantillons par des tubes à décharge alimentés par une batterie de capacités :

- énergie des éclairs : de l'ordre de 60 J
- durée des éclairs : 50 µs.

On observe que les valeurs de $\lambda_{max}$ des composés selon l'invention sont nettement plus élevées que pour le composé 7 de l'art antérieur pris comme référence et que pour les composés de la demande JP 3251587.

## II. ETUDE COMPARATIVE ENTRE UN COMPOSE DE L'INVENTION ET UN HOMOLOGUE NON SUBSTITUE EN 6' DE L'ART ANTERIEUR

### 1. Etude de la cinétique photochromique et du spectre d'absorption dans le visible de la forme ouverte, dans une matrice polymère.

On compare le composé photochromique A de l'exemple 1, de formule :

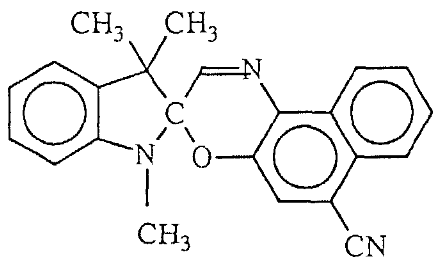

(A)

au composé B non substitué en 6' de l'art antérieur, de formule :

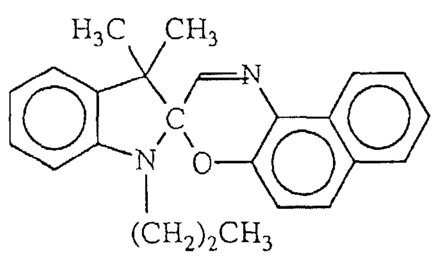

(B)

Ces composés sont intégrés dans un vernis polyuréthane thermodurcissable à raison de 5% en poids et appliqués sur un verre en polymère de di(allylcarbonate) de diéthylèneglycol (verre ORMA®).

Le revêtement photochromique est d'une épaisseur de l'ordre de 15 µm.

Les revêtements photochromiques sont irradiés par une lampe Xenon 150 W, 0,5 $mW/cm^2$, 13 Klux pendant 15 minutes (phase de coloration du vernis photochromique). L'irradiation est alors stoppée. Il se produit une phase de décoloration.

On mesure la variation du pourcentage de transmission du vernis photochromique en fonction du temps pendant les deux phases en se plaçant à la longueur d'onde $\lambda_{max}$ correspondant à l'absorption maximale de chaque composé.

On enregistre également le spectre d'absorption dans le visible de la forme ouverte de chaque composé intégré dans le vernis.

L'étude cinétique du composé A a été réalisée à 35°C à la longueur d'onde correspondant au maximum d'absorption $\lambda_{maX}$ = 650nm.

Les résultats sont indiqués dans le tableau 1 et dans la figure 1.

Les spectres d'absorption dans le visible du vernis photochromique contenant le composé A ont été effectués à 20 et 35°C et sont représentés dans les figures 3 et 4.

L'étude cinétique du composé B a été réalisée à 20 et 35°C avec $\lambda_{max}$ = 610 nm.

Les résultats à 20°C sont indiqués dans le tableau 2 et la figure 2.

Le spectre d'absorption dans le visible du vernis contenant le composé B a été effectué à 20°C et est représenté dans la figure 5.

**RESULTATS**

α. CINETIQUE PHOTOCHROMIQUE DU COMPOSE A
A 35°C ET $\lambda_{max}$ = 650 nm.

TABLEAU 1

| Temps en mn | Temps en s | % de transmission | D.O. |
|---|---|---|---|
| 0 | 0 | 92,1 | 0,036 |
| 0,5 | 30 | 51,4 | 0,289 |
| 1 | 60 | 41,1 | 0,386 |
| 2 | 120 | 31,4 | 0,503 |
| 5 | 300 | 18,3 | 0,738 |
| 10 | 600 | 11,5 | 0,939 |
| 15 | 900 | 10 | 1,000 |
| 15,5 | 930 | 27,8 | 0,556 |
| 16 | 960 | 34,8 | 0,458 |
| 17 | 1020 | 56,7 | 0,246 |
| 20 | 1200 | 83,5 | 0,078 |
| 25 | 1500 | 90 | 0,046 |
| 35 | 2100 | 91,1 | 0,040 |

β. CINETIQUE PHOTOCHROMIQUE DU COMPOSE B
A 20°C ET $\lambda_{max}$ = 610 nm.

TABLEAU 2

| Temps en mn | Temps en s | % de transmission | D.O. |
|---|---|---|---|
| 0 | 0 | 91,6 | 0,038 |
| 0,5 | 30 | 79,9 | 0,097 |
| 1 | 60 | 75,6 | 0,121 |
| 2 | 120 | 71,2 | 0,148 |
| 5 | 300 | 65,5 | 0,184 |
| 10 | 600 | 63 | 0,201 |
| 15 | 900 | 61,4 | 0,212 |
| 15,5 | 930 | 70,2 | 0,154 |
| 16 | 960 | 74 | 0,131 |
| 17 | 1020 | 77,6 | 0,110 |
| 20 | 1200 | 82,3 | 0,085 |
| 25 | 1500 | 85,5 | 0,068 |
| 35 | 2100 | 88,2 | 0,055 |
| 45 | 2700 | 89,3 | 0,049 |

γ. CINETIQUE PHOTOCHROMIQUE DU COMPOSE B
A 35°C ET $\lambda_{max}$ = 610 nm.

Après 15 minutes d'irradiation, on observe un pourcentage de transmission de 85-86% alors qu'à l'état initial non excité, la transmission est de 90%. Les performances photochromiques du composé B sont très faibles à 35°C.

**INTERPRETATION DES RESULTATS** :

Le spectre d'absorption dans le visible du composé B à 20°C, représenté dans la figure 5, montre que la colorabilité du composé B est très faible.

Il résulte des tableaux 1 et 2 et des figures 1 et 2 relatifs à l'étude de la cinétique photochromique des composés A et B, après 15 minutes d'irradiation UV, que :

- le composé B de l'art antérieur, sous forme ouverte, conduit, à 20°C, à une coloration de faible intensité (Densité optique : D.O. = 0,212);
- tandis que le composé A atteint très rapidement, à une température de 35°C, une coloration 5 fois plus intense (D.O. = 1,00).

Les figures 3, 4 et 5 relatives aux spectres d'absorption dans le visible des composés A et B après 15 minutes d'irradiation, confirment le fait que le composé A, sous forme ouverte, présente une colorabilité d'amplitude plus élevée que le composé B, à 20°C.

Ils montrent également qu'à 35°C, le composé A présente une forte coloration au maximum d'absorption ($\lambda_{max}$).

**2. Etude du spectre UV d'absorption de la forme fermée**

On compare le composé photochromique A de l'exemple 1 avec le composé homologue C non substitué en 6', de formule :

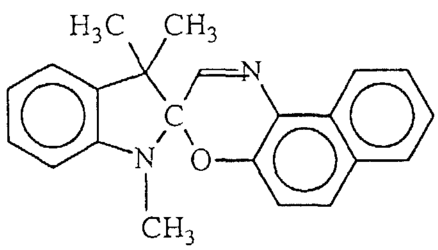

(C)

On mesure les spectres d'absorption de la forme fermée des composés A et C en solution dans l'acétonitrile. Une comparaison des deux spectres est présentée en figure 6.

Il résulte de cette figure que le composé A présente un spectre UV d'absorption de la forme fermée (non excité), dans lequel on observe un déplacement des bandes d'absorption vers des longueurs d'onde plus élevées et des maxima d'absorption pour les longueurs d'onde de valeur élevée, dont l'amplitude est sensiblement plus importante, par rapport aux bandes du spectre UV d'absorption du composé B sous sa forme fermée.

EXEMPLE 8

5-acryloxy-6'-cyano-1,3,3-triméthyl-spiro-[indoline-2,3'-[3H]-napht-[2,1-b]-[1,4]-oxazine] :

Etape 1

6'-cyano-5-hydroxy-1,3,3-triméthyl-spiro-[indoline-2,3'-[3H]-napht-[2,1-b]-[1,4]-oxazine]

Obtenue par réaction de la 4-cyano-2-hydroxynaphtylamine (V) sur la 5-hydroxy-2-méthylène-1,3,3-triméthylindolènine.

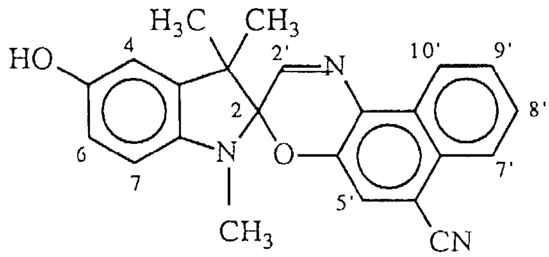

Rendement : 30%

Point de fusion : 205-206°C.

RMN $^1H$ (250 MHz, acétone-d6, TMS), δppm : 1,31 (3H, s, 3-$CH_3$); 1,37 (3H, s, 3-$CH_3$); 2,69 (3H, s, N-$CH_3$); 6,49 (1H, d, 7-H); 6,68 (1H, dd, 6-H); 6,71 (1H, d, 4-H); 7,65-7,79 (3H, m, 5',8' et 9'-H); 7,82 (1H, s, 2'-H); 8,01 (1H, s,OH); 8,09 (1H, d, 7'-H); 8,69 (1H, d, 10'-H).

Etape 2

On ajoute goutte à goutte 0,15 g de chlorure d'acide acrylique à la solution refroidie (0°C) de 0,1 g de SPO obtenue à l'étape précédente et 0,3 g de triéthylamine dans 50 ml de $CH_2Cl_2$.

On laisse agir 2 heures à température ambiante. L'évaporation du solvant et la purification par chromatographie flash (85 hexane/15 acétate d'éthyle) donne le produit recherché.

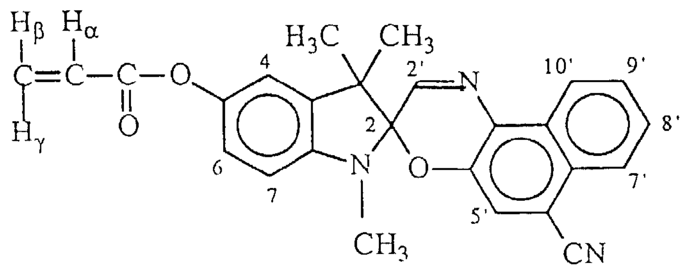

Rendement : 79%

Point de fusion : 135-136°C.

RMN $^1H$ (250 MHz, $CDCl_3$, TMS), δppm : 1,32 (3H, s, 3-$CH_3$); 1,39 (3H, s, 3-$CH_3$); 2,74 (3H, s, N-$CH_3$); 6,01 (1H, d, Hβ); 6,33 (1H, dd, Hα); 6,51 (1H, d, 7-H); 6,61 (1H, d, Hγ); 6,87 (1H, d, 4-H) ); 6,98 (1H, s, 5'-H); 7,61 (1H, dd, 8'-H) ; 7,70 (1H, dd, 9'-H); 7,88 (1H, s, 2'-H); 8,14 (1H, d, 7'-H); 8,66 (1H, d, 10'-H).

Forme ouverte : $\lambda_{max}$ (toluène) : 646 nm

EXEMPLE 9

6'-p-tolylsulfonyl-1,3,3-triméthyl-spiro-[indoline-2,3'-[3H]-napht-[2,1-b]-[1,4]oxazine] :

**A. Synthèse de la 4-p-tolylsulfonyl-2-hydroxynaphtylamine**

Cette synthèse est effectuée selon le schéma réactionnel suivant :

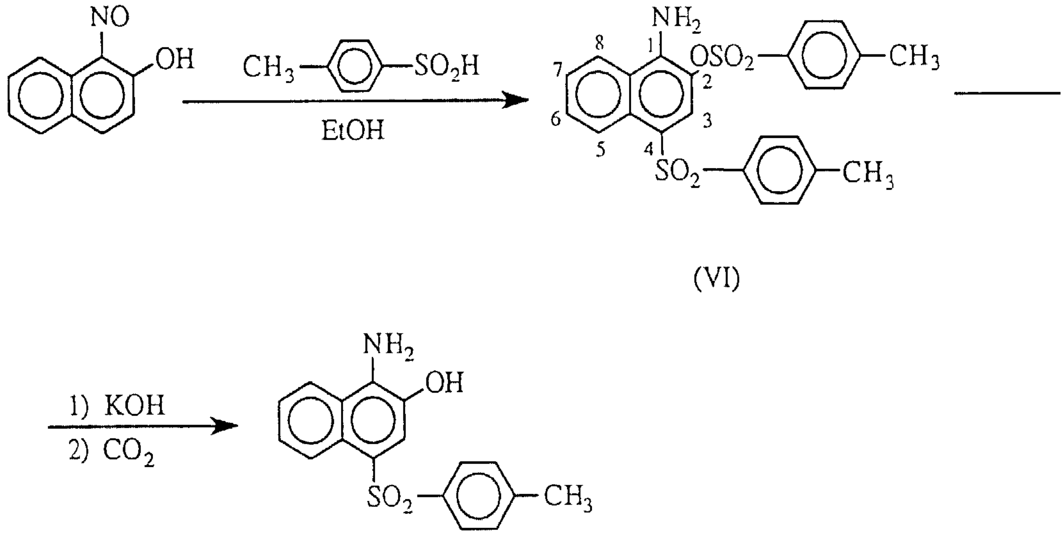


(VI)

(VII)

On ajoute 10 g (0,058 moles) de 1-nitroso-2-naphtol à une solution refroidie d'acide p-toluenesulfinique (27 g, 0,173 moles) dans 50 ml d'éthanol. On chauffe à reflux 1 heure, on refroidit et on laisse le produit précipiter à froid pendant 12 heures. Après filtration et deux cristallisations dans l'éthanol, on obtient 9,2 g (34 %) de composé (VI) sous forme de cristaux blancs.
Point de fusion : 151-152°C
RMN $^1H$ (250 MHz, CDCl3, TMS), δppm : 2,34 (3H, s, $CH_3$); 2,49 (3H, s, $CH_3$); 5,06 (2H, s. large, $NH_2$); 7,20-7,86 (12H, m, arom.H); 8,5 (1H, d, 8-H).

On mélange une solution de 3,4 g (7,25 mmoles) de tosylate (VI) dans 20 ml d'éthanol avec une solution de 3,4 g (60,7 mmoles) d'hydroxyde de potassium dans 40 ml d'eau. On porte à reflux 30 minutes, on refroidit, on laisse une nuit à froid et on filtre. On sature le filtrat avec du gaz carbonique, on filtre le précipité et on le sèche.
On obtient 1,4 g (62 %) de 4-p-tolylsulfonyl-2-hydroxynaphtylamine (VII) qu'on utilise dans la synthèse de SPO sans purification supplémentaire.
Point de fusion : 181-184°C.

**B. Synthèse de la spironaphtoxazine correspondante :**

La spirooxazine est obtenue par réaction de la 4-p-tolylsulfonyl-2-hydroxynaphtylamine (VII) sur la 2-méthylène-1,3,3-triméthylindolénine. Les conditions réactionnelles et la purification sont identiques à l'exemple 1.

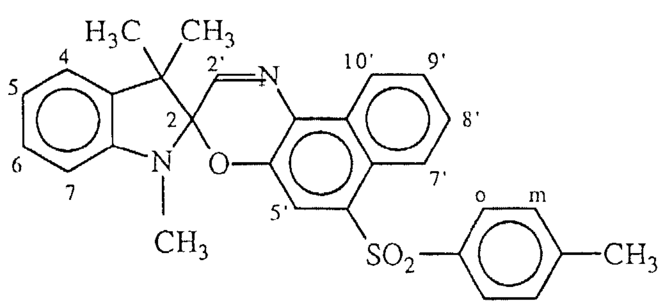

Rendement : 10 %
Point de fusion : 204-205°C
RMN $^1H$ (250 MHz, $CDCl_3$, TMS), δppm : 1,34 (3H, s, 3-$CH_3$); 1,35 (3H, s, 3-$CH_3$); 2,39 (3H, s, p-$CH_3$); 2,73 (3H, s,

$N-CH_3$); 6,58 (1H, d, 7-H); 6,91 (1H, dd, 5-H); 7,08 (1H, d, 4-H) 7,23 (3H, m,m-H, 6-H); 7,46 (1H, dd, 8'-H); 7,58 (1H, dd, 9'-H); 7,82 (2H, d, o-H) ; 7,87 (1H, s, 2'-H); 8,02 (1H, s, 5'-H); 8,57 (1H, d, 7'-H); 8,66 (1H, d, 10'-H).
Forme ouverte : $\lambda_{max}$ (toluène) : 632 nm

EXEMPLE 10

6'-Phénylsulfonyl-1,3,3-triméthyl-spiro-[indoline-2,3'-[3H]-napht-[2,1-b]-[1,4]oxazine] :

**A. Synthèse de la 4-phénylsulfonyl-2-hydroxynaphtylamine**

La 4-phénylsulfonyl-2-hydroxynaphtylamine (VIII) est obtenue selon la méthode décrite pour l'obtention de (VII) (exemple 9) à partir de 1-nitroso-2-naphtol et d'acide benzenesulfinique.

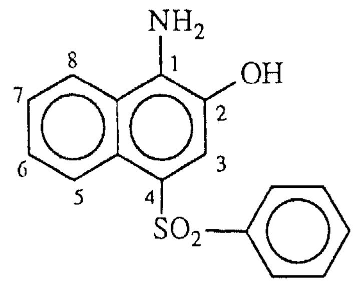

(VIII)

Rendement: 16 %
Point de fusion : 176-177°C

**B. Synthèse de la spironaphtoxazine correspondante :**

Elle est obtenue par réaction de la 4-phénylsulfo-2-hydroxynaphtylamine sur la 2-méthylène-1,3,3-triméthylindolénine.

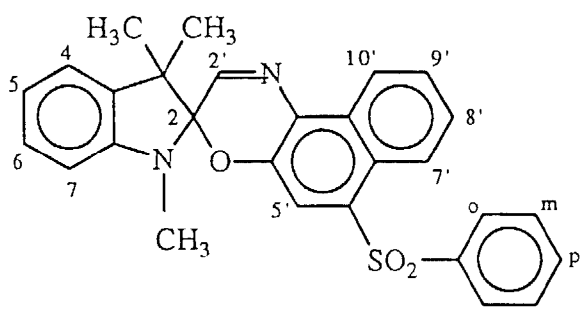

Rendement : 21 %
Point de fusion : 195-196°C
RMN $^1H$ (250 MHz, $CDCl_3$, TMS), δppm : 1,36 (3H, s, $3-CH_3$); 1,37 (3H, s, $3-CH_3$); 2,75 (3H, s, $N-CH_3$); 6,59 (1H, d, 7-H); 6,93 (1H, dd, 5H); 7,10 (1H, d, 4-H); 7,24 (1H, dd, 6-H), 7,43-7,53 (4H, m, 8'-H, p-H,m-H); 7,60 (1H, dd, 9'-H); 7,89 (1H, s, 2'-H); 7,94 (2H, d, o-H); 8,08 (1H,s, 5'-H) ; 8,54 (1H, d, 7'-H); 8,66 (1H, d, 10'-H).
RMN$^{13C}$ (62,5 MHz, $CDCl_3$, TMS), δppm : 21,0 (q, $3-CH_3$); 25,5(q, $3-CH_3$); 29,8 (q, $N-CH_3$); 52,2 (s, 3-C); 99,1 (s, 2-C); 107,4 (d, 7-C); 120,4 (d, 5-C); 121,2 (d, 5'-C); 121,6 (d, 4-C); 122,8 (d, 10'-C); 124,4 (d, 7'-C) ; 126,4 (d, 8'-C); 127,6 (d, o-C); 127,9 (d, 9'-C); 128,3 (d, 6-C); 129,3 (d, m-C); 133,4 (d, p-C); 154,6 (d, 2'-C).
Forme ouverte : $\lambda_{max}$ (toluène) : 642 nm

EXEMPLE 11

5-méthoxy-6'-phénylsulfonyl-1,3,3-triméthyl-spiro-[indoline-2,3'-[3H]-napht-[2,1-b]-[1,4]oxazine] :

Elle est obtenue par réaction de la 4-phénylsulfo-2-hydroxynaphtylamine sur la 5-méthoxy-2-méthylène-1,3,3-triméthylindolénine.

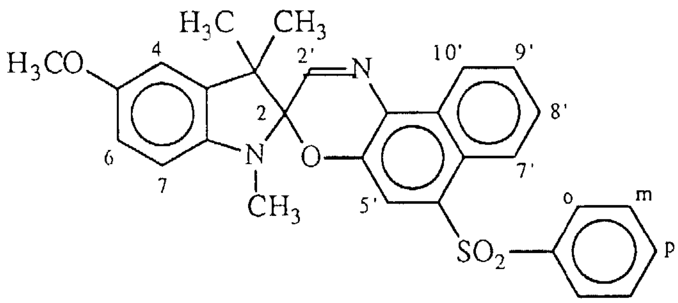

Rendement : 14 %
Point de fusion : 161-162°C
RMN $^1H$ (250 MHz, $CDCl_3$, TMS), δppm : 1,34 (3H, s, 3-$CH_3$) ; 1,38 (3H, s, 3-$CH_3$) ; 2,69 (3H, s, N-$CH_3$) ; 3,81 (3H, s, O-$CH_3$) ; 6,50 (1H, d, 7H) ; 6,74-6,77 (2H, m, 6-H, 4-H) ; 7,35-7,51 (4H, m, 8'-H, p-H, m-H) ; 7,55 (1H, dd, 9'H) ; 7,88 (1H, s, 2'-H) ; 7,93 (2H, dd, o-H) ; 8,10 (1H, s, 5'-H) ; 8,53 (1H, d, 7'-H) ; 8,53 (1H, d, 7'-H) ; 8,66 (1H, s, 10'-H).
Forme ouverte : $\lambda_{max}$ (toluène) : 657 nm

EXEMPLE 12

5-acryloxy-6'-phénylsulfonyl-1,3,3-triméthylspiro[indoline-2,3'-[3H]-napht[2,1-b]-[1,4]-oxazine] ;

Etape 1

5-hydroxy-6'-phénylsulfonyl-1,3,3,-triméthylspiro[indoline-2,3'-[3H]-napht[2,1-b]-[1,4]-oxazine] :
Obtenue par réaction de la 4-phénylsulfo-2-hydroxynaphtylamine sur la 5-hydroxy-2-méthylène-1,3,3-triméthylindolénine

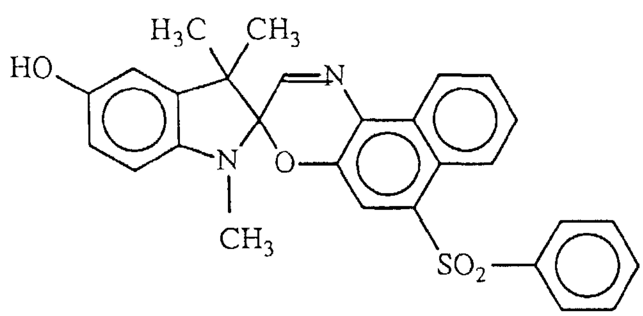

Rendement : 28 %

Etape 2

On traite 0,1 g de SPO obtenue à l'étape précédente par 0,15 g de chlorure d'acide acrylique, comme dans l'exemple 8, étape 2. La purification par chromatographie flash (85 hexane/15 acétate d'éthyle) donne le produit recherché.

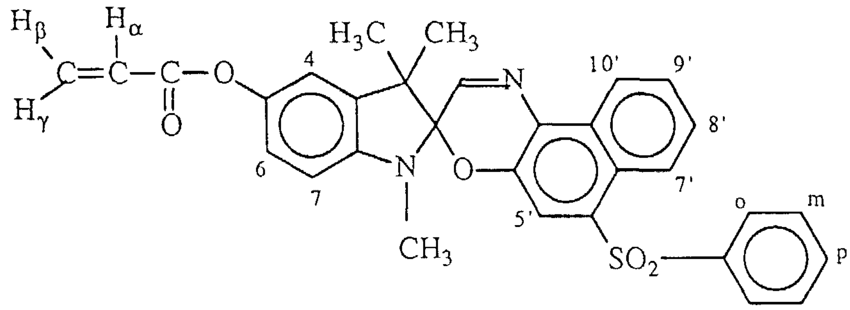

Rendement: 68 %
Point de fusion : 183-184°C
RMN $^1H$(250 MHz, $CDCl_3$, TMS), δppm ; 1,34 (3H, s, 3-$CH_3$) ; 1,39 (3H, s, 3-$CH_3$) ; 2,74 (3H, s, N-$CH_3$) ; 6,01 (1H, d, Hβ) ; 6,33 (1H, dd, Hα) ; 6,56 (1H, d, 7H) ; 6,61 (1H, d, Hγ) ; 6,89 (1H, d, 4-H) ; 6,97 (1H, dd, 6-H) ; 7,45-7,64 (5H, m, 8'-H, 9'-H, p-H, m-H) ; 7,88 (1H, s, 2'-H) ; 7,96 (2H, d, o-H) ; 8,07 (1H, s, 5'-H) ; 8,56 (2H, d, 7'-H) ; 8,66 (1H, d, 10'-H)
Forme ouverte : $\lambda_{max}$ (toluène) : 643 nm

EXEMPLE 13

En suivant le schéma général de synthèse, on synthétise le composé

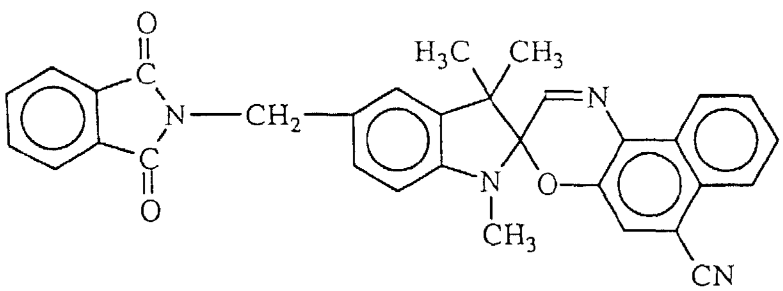

Ce composé peut être synthétisé par le schéma réactionnel mentionné précédemment en utilisant la base de Fischer 1,3,3-triméthyl-2-méthylène-5-N-phtalimidométhyl indoline. Cette base de Fischer ainsi que le procédé de préparation sont décrits dans Australian Journal Chemistry 1977, Vol. 30 pp 689-694.

Propriétés spectrocinétiques des composés des exemples 8 à 12

Les propriétés spectrocinétiques sont mesurées en solution dans le toluène, dans les mêmes conditions que pour les exemples 1 à 7.

| Exemples | $\lambda_{max}$ (nm) | Constante cinétique de décoloration thermique $K_{\Delta}$ ($s^{-1}$) | Ao Colorabilité |
|---|---|---|---|
| 8 | 646 | 0,17 | 0,77 |
| 9 | 632 | 0,15 | 1,32 |
| 10 | 642 | 0,13 | 1,17 |
| 11 | 657 | 0,20 | |
| 12 | 643 | 0,18 | 0,95 |

On observe que les valeurs de $\lambda_{max}$ des composés 8 à 12 sont élevées.

Exemples d'utilisation des composés photochromiques de l'invention

**1) Lentilles de contact souples hydrophiles photochromiques**

On prépare des lentilles de contact hydrophiles en copolymère MMA (méthacrylate de méthyle) et NVP (N-vinylpyrrolidone) dans des proportions 72,8 % et 26,1 %, réticulé par l'allylméthacrylate 0,23 %.

La lentille est obtenue en mélangeant la N-vinylpyrrolidone avec le MMA, un amorceur thermique l'AIBN (Azobisisobutyronitrile) (0,89 %), le composé photochromique 0,1 % en mole, le réticulant, puis la composition obtenue est polymérisée thermiquement pendant 33 heures, la température partant de 40°C pour atteindre 120°C en fin de cycle.

Chacun des composés photochromiques des exemples 1, 2, 3, 4, 6, 8, 9, 12 et 13 est incorporé séparément dans les lentilles de contact hydrophiles MMA/NVP, conformément au mode opératoire précédent.

Exposés à la lumière solaire, les lentilles initialement incolores prennent une coloration bleu-vert, qui disparaît lorsque l'irradiation cesse.

On note que la coloration apparaît plus intense dans le cas des lentilles incluant les composés photochromiques des exemples 3, 4 et 8.

**2) Lentilles de contact rigides photochromiques**

On prépare des lentilles de contact rigides en PMMA (polyméthacrylate de méthyle) par polymérisation d'un mélange renfermant 100 parties en poids de méthacrylate de méthyle (MMA), 2 parties en poids de DMEG (diméthacrylate d'éthylèneglycol), et 0,03 parties en poids d'AIBN.

Le composé photochromique de l'exemple 8 est incorporé dans le mélange polymérisable à raison de 0,1 % molaire.

Le mélange est introduit dans un tube en polypropylène puis la polymérisation est effectuée thermiquement par chauffage pendant 15 heures à 64°C puis pendant 8 heures à 83°C.

Après démoulage, on taille et l'on usine les lentilles de contact.

Exposées à la lumière solaire, les lentilles initialement incolores prennent une coloration bleu-vert.

Le photochromisme de ces lentilles est particulièrement persistant dans le temps.

## Revendications

1. Composés photochromiques de structure spiro[indoline-[2,3']-benzoxazine], caractérisés par le fait qu'ils comportent en position 6' un groupement $R_6$ choisi parmi les groupements suivants : cyano, phénylsulfonyl lié par l'atome de souffre au carbone 6' et un cycle benzénique condensé en 7',8' au noyau benzoxazine.

2. Composés photochromiques selon la revendication 1, caractérisés par le fait qu'ils possèdent une structure spiro [indoline-2,3'-[3H]-napht[2,1,b]-[1,4]-oxazine].

3. Composés photochromiques selon la revendication 1, caractérisés par le fait qu'ils répondent à la formule suivante :

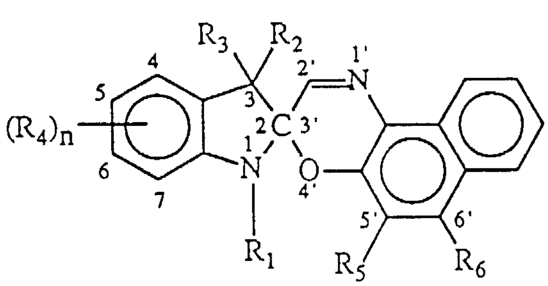

(I)

dans laquelle :

n varie de 0 à 4;

- $R_1$ représente :

  i) un groupe alkyle de 1 à 16 atomes de carbone;
  ii) un groupe allyle, phényle, arylalkyle mono- ou disubstitué par des substituants du type alkyle ou alcoxy de 1 à 6 atomes de carbone ou des atomes d'halogène tels que chlore;
  iii) un groupe alicyclique éventuellement substitué;
  iv) un groupe hydrocarbure aliphatique comportant dans sa chaîne un ou plusieurs hétéroatomes tels que O, N ou S, et éventuellement une fonction acide, ester ou alcool;

- $R_2$ et $R_3$ peuvent chacun et indépendamment l'un de l'autre représenter un groupe $C_1$-$C_8$ alkyle, phényle, phényle mono- ou disubstitué par des groupes $C_1$-$C_4$ alkyle et/ou $C_1$-$C_5$ alcoxy, ou peuvent être combinés pour former une chaîne cyclique de 6 à 8 atomes de carbone;
- $R_4$ et $R_5$ représentent, indépendamment l'un de l'autre :

  i) un atome d'hydrogène, une fonction amine NR'R", où R' et R" représentent chacun et indépendamment un atome d'hydrogène, un groupe alkyle, cycloalkyle, phényle ou un dérivé substitué de celui-ci; R' et R" peuvent se combiner pour former un cycloalkyle pouvant être substitué et contenir un ou plusieurs hétéroatomes;
  ii) un groupe R, OR, SR, COR ou COOR, dans lequel R représente un atome d'hydrogène, un groupe alkyle de 1 à 6 atomes de carbone ou un groupe aryle ou hétéroaryle;
  iii) un atome d'halogène, un groupe $C_1$-$C_4$ monohaloalkyle, ou un groupe $C_1$-$C_4$ polyhaloalkyle;
  iv) -$NO_2$, CN, SCN,
  chacun des substituants $R_4$ pouvant être présent sur l'un quelconque des atomes de carbone convenables de la partie indoline du composé photochromique, en positions 4, 5, 6 et 7,
  v) un groupement

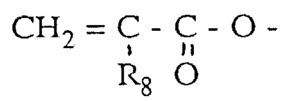

  où $R_8$ désigne H ou $CH_3$;
  vi) un groupement

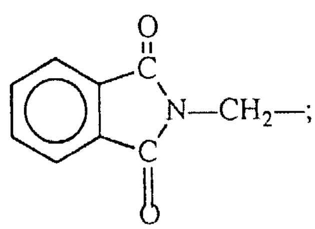

  $R_6$ est choisi parmi les groupements CN et de formule

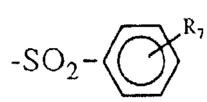

dans laquelle $R_7$ désigne un atome d'hydrogène, un alkyle ayant de 1 à 6 atomes de carbone, un alcoxy ayant de 1 à 6 atomes de carbone, ou un atome d'halogène.

**4.** Composés selon la revendication 3, caractérisé par le fait qu'ils répondent à la formule suivante :

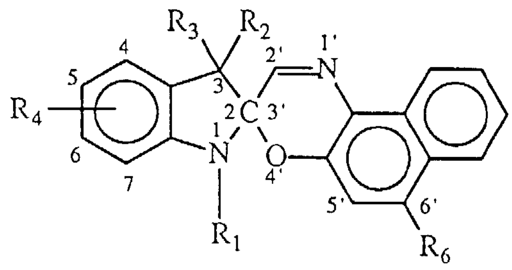

(I')

dans laquelle $R_1$ désigne un groupement alkyle ayant de 1 à 16 atomes de carbone ou un groupement allyle ;
$R_2$ et $R_3$ représentent chacun et indépendamment l'un de l'autre un groupement alkyle ayant de 1 à 8 atomes de carbone ;
$R_4$ désigne un atome d'halogène, un atome d'hydrogène ou un radical alcoxy, un groupement

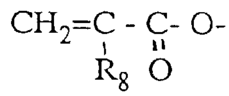

dans lequel $R_8$ représente H ou $CH_3$; et
$R_6$ a la même signification que dans la revendication 3.

**5.** Composés selon la revendication 4, caractérisés par le fait qu'ils répondent à la formule suivante :

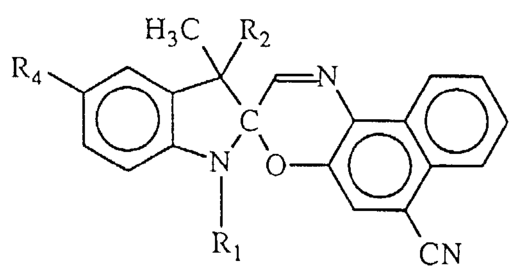

(I")

dans laquelle :

$R_1$ désigne un alkyle ayant de 1 à 16 atomes de carbone ou un groupe allyle;
$R_2$ désigne un alkyle ayant de 1 à 8 atomes de carbone;
$R_4$ désigne un atome d'halogène, un atome d'hydrogène, un radical alcoxy ayant de 1 à 6 atomes de carbone ou un radical

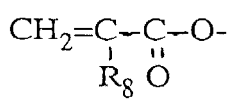

avec $R_8$ désignant H ou $CH_3$.

**6.** Composés selon la revendication 5, caractérisés par le fait que dans la formule (I") :

$R_1$ désigne un radical méthyle, hexadécyle ou allyle;
$R_2$ désigne un radical méthyle ou éthyle;
$R_4$ désigne un atome d'hydrogène, un atome de chlore ou un radical méthoxy, ou $CH_2$=CH-COO-.

**7.** Procédé de préparation des composés tels que définis dans l'une quelconque des revendications 1 à 6, caractérisé par le fait qu'il comprend la condensation d'une base de Fischer avec un orthoaminophénol substitué en position 4 par un groupement $R_6$ dans un milieu solvant en présence d'un agent oxydant.

**8.** Procédé de préparation selon la revendication 7, caractérisé par le fait qu'il comprend la condensation d'une base de Fischer, de formule :

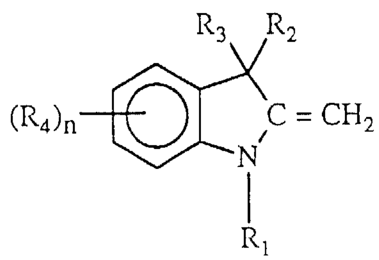

(II)

dans laquelle $R_1$, $R_2$, $R_3$, $R_4$ et n ont les mêmes significations indiquées dans la revendication 1, avec un orthoaminophénol annélé de formule :

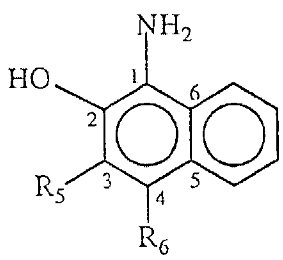

(III)

où $R_5$ et $R_6$ ont les mêmes significations indiquées dans la revendication 3, dans un milieu solvant en présence d'un agent oxydant.

**9.** Utilisation d'un composé selon l'une quelconque des revendications 1 à 6, comme composé photochromique dans l'optique ophtalmique.

**10.** Composition destinée à être appliquée sur ou introduite dans un matériau polymère organique transparent, caractérisée par le fait qu'elle contient au moins un composé photochromique tel que défini dans l'une quelconque des revendications 1 à 6, dans des quantités suffisantes pour permettre au matériau exposé à une radiation ultraviolette de changer de couleur.

**11.** Composition selon la revendication 10, caractérisée par le fait que la composition est sous forme liquide contenant sous forme dissoute ou dispersée, les composés photochromiques selon l'une quelconque des revendications 1 à 6, dans un milieu à base de solvants appropriés pour être appliqués ou introduits dans un matériau polymère transparent.

**12.** Composition destinée à être appliquée sur ou introduite dans un matériau polymère organique transparent, caractérisée par le fait qu'elle est constituée par une solution incolore ou transparente à base de polymères, de copolymères ou de mélange de polymères transparents dans un solvant organique approprié, contenant au moins un composé photochromique tel que défini dans l'une quelconque des revendications 1 à 6, dans des quantité suffisantes pour permettre au matériau exposé à une radiation ultraviolette de changer de couleur.

**13.** Matériau solide transparent approprié pour réaliser des lentilles ophtalmiques, caractérisé par le fait qu'il comporte sur la surface et/ou à l'intérieur au moins un composé photochromique tel que défini dans l'une quelconque des revendications 1 à 6, dans des quantités suffisantes pour permettre au matériau exposé à une radiation ultraviolette de changer de couleur.

**14.** Matériau solide transparent selon la revendication 13, caractérisé par le fait qu'il contient de 0,07 à 20% en poids de composés photochromiques.

**15.** Composition ou matériau solide transparent, selon l'une quelconque des revendications 10 à 14, caractérisée par le fait que le composé photochromique tel que défini dans l'une quelconque des revendications 1 à 6, est utilisé conjointement avec d'autres composés photochromiques donnant lieu à des colorations différentes.

**16.** Vernis de transfert, caractérisé par le fait qu'il contient au moins un composé tel que défini dans l'une quelconque des revendications 1 à 6.

**Claims**

**1.** Photochromic compounds of spiro[indoline[2,3']-benzoxazine] structure, characterized in that they contain, in the 6' position, a group $R_6$ chosen from the following groups: cyano, phenylsulfonyl linked via the sulfur atom to carbon 6' and a 7',8'-fused benzene ring on the benzoxazine nucleus.

**2.** Photochromic compounds according to claim 1, characterized in that they possess a spiro[indoline-2,3'[3H]naphth [2,1,b][1,4]oxazine].

**3.** Photochromic compounds according to claim 1, characterized in that they correspond to the following formula:

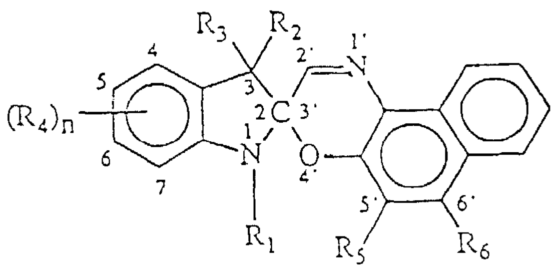

(I)

in which:

n ranges from 0 to 4;

$R_1$ represents:

i) an alkyl group of 1 to 16 carbon atoms;
ii) an allyl group, a phenyl group, an arylalkyl group mono- or disubstituted with substituents of the alkyl or alkoxy type of 1 to 6 carbon atoms or halogen atoms such as chlorine;
iii) an optionally substituted alicyclic group;
iv) an aliphatic hydrocarbon group containing one or more hetero atoms such as O, N or S in its chain, and optionally an acid, ester or alcohol function;

$R_2$ and $R_3$ may, independently of each other, represent a $C_1$-$C_8$ alkyl group, a phenyl group, a phenyl group mono-or disubstituted with $C_1$-$C_4$ alkyl and/or $C_1$-$C_5$ alkoxy groups, or may be combined to form a cyclic chain of 6 to 8 carbon atoms;
$R_4$ and $R_5$ represent, independently of each other:

i) a hydrogen atom, an amine function NR'R", where R' and R" each independently represent a hydrogen atom, an alkyl, cycloalkyl or phenyl group or a substituted derivative thereof; R' and R" may combine to form a cycloalkyl which may be substituted and may contain one or more hetero atoms;
ii) a group R, OR, SR, COR or COOR, in which R represents a hydrogen atom, an alkyl group of 1 to 6 carbon atoms or an aryl or heteroaryl group;
iii) a halogen atom, a $C_1$-$C_4$ monohaloalkyl group, or a $C_1$-$C_4$ polyhaloalkyl group;

iv) $-NO_2$ CN, SCN,
it being possible for each of the substituents $R_4$ to be present on any of the suitable carbon atoms of the indoline part of the photochromic compound, in positions 4, 5, 6 and 7,
v) a group

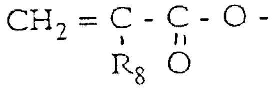

with $R_8$ representing H or $CH_3$;
vi) a group

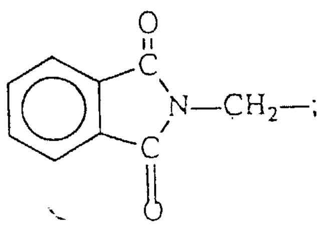

$R_6$ is chosen from groups CN,

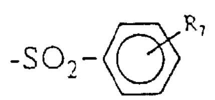

in which formula $R_7$ denotes a hydrogen atom, an alkyl having from 1 to 6 carbon atoms, an alkoxy having from 1 to 6 carbon atoms or a halogen atom.

**4.** Compounds according to claim 3, characterized in that they correspond to the following formula:

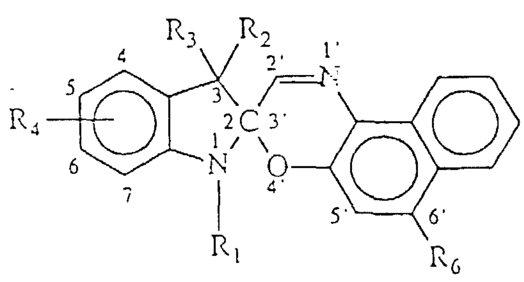

(I')

in which $R_1$ denotes an alkyl group having from 1 to 16 carbon atoms or an allyl group;
$R_2$ and $R_3$ represent, independently of each other, an alkyl group having from 1 to 8 carbon atoms;
$R_4$ denotes a halogen atom, a hydrogen atom, an alkoxy radical or a group

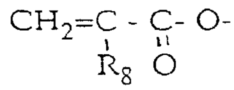

in which $R_8$ represents H or $CH_3$: and
$R_6$ has the same meaning as in claim 3.

**5.** Compounds according to claim 4, characterized in that they correspond to the following formula:

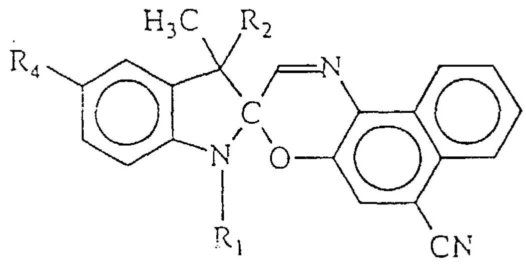 (I")

in which:

$R_1$ denotes an alkyl having from 1 to 16 carbon atoms or an allyl group;
$R_2$ denotes an alkyl having from 1 to 8 carbon atoms;
$R_4$ denotes a halogen atom, a hydrogen atom, an alkoxy radical having from 1 to 6 carbon atoms or a radical

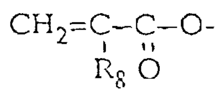

with $R_8$ denoting H or $CH_3$.

**6.** Compounds according to claim 5, characterized in that, in formula (I"):

$R_1$ denotes a methyl, hexadecyl or allyl radical;
$R_2$ denotes a methyl or ethyl radical;
$R_4$ denotes a hydrogen atom, a chlorine atom, a methoxy radical or a radical $CH_2$=CH-COO-.

**7.** Process for the preparation of the compounds as defined in any one of claims 1 to 6, characterized in that it comprises the condensation of a Fischer base with an ortho-aminophenol substituted in position 4 with a group $R_6$ in a solvent medium in the presence of an oxidizing agent.

**8.** Preparation process according to claim 7, characterized in that it comprises the condensation of a Fischer base, of formula:

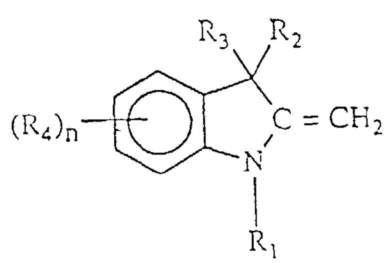 (II)

in which $R_1$, $R_2$, $R_3$, $R_4$ and n have the same meanings indicated in claim 1,

with an annellated ortho-aminophenol of formula:

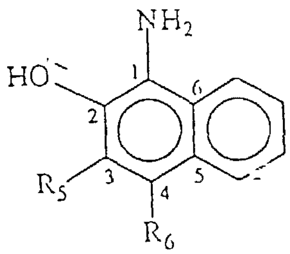

(III)

where $R_5$ and $R_6$ have the same meanings indicated in claim 3,in a solvent medium in the presence of an oxidizing agent.

**9.** Use of a compound according to any one of claims 1 to 6, as a photochromic compound in ophthalmic optics.

**10.** Composition intended to be applied to or introduced into a transparent organic polymer, characterized in that it contains at least one photochromic compound as defined in any one of claims 1 to 6, in amounts which are sufficient to allow the material exposed to ultraviolet radiation to change color.

**11.** Composition according to claim 10, characterized in that the composition is in liquid form containing, in dissolved or dispersed form, the photochromic compounds according to any one of claims 1 to 6, in a medium based on solvents which are suitable to be applied or to be introduced into a transparent polymer.

**12.** Composition intended to be applied to or introduced into a transparent organic polymer, characterized in that it consists of a colorless or transparent solution based on polymers, on copolymers or on a mixture of transparent polymers in a suitable organic solvent, containing at least one photochromic compound as defined in any one of claims 1 to 6, in amounts which are sufficient to allow the material exposed to ultraviolet radiation to change color.

**13.** Transparent solid material which is suitable for producing ophthalmic lenses, characterized in that it contains, on the surface and/or inside, at least one photochromic compound as defined in any one of claims 1 to 6, in amounts which are sufficient to allow the material exposed to ultraviolet radiation to change color.

**14.** Transparent solid material according to claim 13, characterized in that it contains from 0.07 to 20% by weight of photochromic compounds.

**15.** Transparent solid material or composition according to any one of claims 10 to 14, characterized in that the photochromic compound as defined in any one of claims 1 to 6 is used in conjunction with other photochromic compounds giving rise to different colorations.

**16.** Transfer varnish, characterized in that it contains at least one compound as defined in any one of claims 1 to 6.

**Patentansprüche**

**1.** Fotochrome Verbindungen mit einer Spiro[indolin-[2,3']-benzoxazin]-Struktur, dadurch gekennzeichnet, daß sie in Position 6' eine Gruppe $R_6$, ausgewählt aus den folgenden Gruppen: Cyano, Phenylsulfonyl, gebunden über das Schwefelatom an den Kohlenstoff 6', und einen in 7', 8' an den Benzoxazinkern kondensierten Benzolring aufweisen.

**2.** Fotochrome Verbindungen nach Anspruch 1, dadurch gekennzeichnet, daß sie eine Spiro[Indolin-2,3'-[3H]-napth [2,1-b]-[1,4]-oxazin]-Struktur aufweisen.

**3.** Fotochrome Verbindungen nach Anspruch 1, dadurch gekennzeichnet, daß sie der folgenden Formel entsprechen:

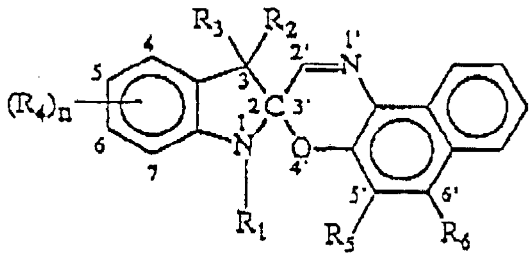

(I)

worin:

n von 0 bis 4 variiert;

- $R_1$ darstellt:

  i) eine Alkylgruppe mit 1 bis 16 Kohlenstoffatomen;
  ii) eine Allyl-, Phenyl-, durch Substituenten vom Alkyl- oder Alkoxytyp mit 1 bis 6 Kohlenstoffatomen oder durch Halogenatome wie Chlor mono- oder disubstituierte Arylalkylgruppe;
  iii) eine gegebenenfalls substituierte alizyklische Gruppe;
  iv) eine aliphatische Kohlenwasserstoffgruppe mit einem oder mehreren Heteroatomen wie O, N oder S und gegebenenfalls einer Säure-, Ester- oder Alkoholfunktion in ihrer Kette;

- $R_2$ und $R_3$ jeweils unabhängig voneinander eine $C_1$-$C_8$-Alkyl-, Phenyl-, durch $C_1$-$C_4$-Alkyl und/oder $C_1$-$C_5$-Alkoxy mono- oder disubstituierte Phenylgruppe darstellen oder zur Bildung einer zyklischen Kette mit 6 bis 8 Kohlenstoffatomen verbunden sein können;
- $R_4$ und $R_5$ unabhängig voneinander darstellen:

  i) ein Wasserstoffatom, eine Aminfunktion NR'R", worin R' und R" jeweils unabhängig voneinander ein Wasserstoffatom, eine Alkyl-, Cycloalkyl-, Phenylgruppe oder ein substituiertes Derivat derselben darstellen; R' und R" können zur Bildung eines Cycloalkyls, welches substituiert sein und ein oder mehrere Heteroatome enthalten kann, verbunden sein;
  ii) eine Gruppe R, OR, SR, COR oder COOR, worin R für ein Wasserstoffatom, eine Alkylgruppe mit 1 bis 6 Kohlenstoffatomen oder eine Aryl- oder Heteroarylgruppe steht;
  iii) ein Halogenatom, eine $C_1$-$C_4$-Monohaloalkylgruppe oder eine $C_1$-$C_4$-Polyhaloalkylgruppe;
  iv) $-NO_2$, CN, SCN,
  wobei jeder der Substituenten $R_4$ an irgendeinem geeigneten Kohlenstoffatom des Indolinteils der fotochromen Verbindung in den Positionen 4, 5, 6 und 7 vorhanden sein kann;
  v) eine Gruppe

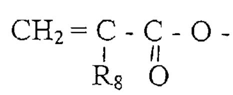

worin $R_8$ für H oder $CH_3$ steht;

  vi) eine Gruppe

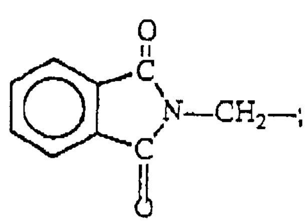

$R_6$ ist ausgewählt aus den Gruppen CN und der Formel

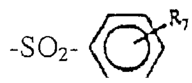

worin $R_7$ ein Wasserstoffatom, ein Alkyl mit 1 bis 6 Kohlenstoffatomen, ein Alkoxy mit 1 bis 6 Kohlenstoffatomen oder ein Halogenatom bezeichnet.

4. Verbindungen nach Anspruch 3, dadurch gekennzeichnet, daß sie der folgenden Formel entsprechen:

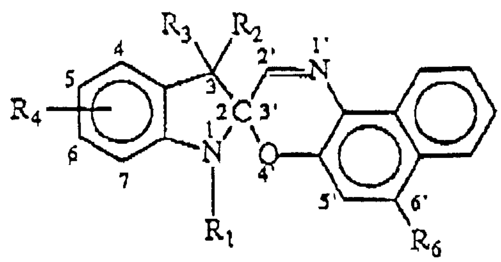

(I')

worin $R_1$ eine Alkylgruppe mit 1 bis 16 Kohlenstoffatomen oder eine Allylgruppe bezeichnet;
$R_2$ und $R_3$ jeweils unabhängig voneinander eine Alkylgruppe mit 1 bis 8 Kohlenstoffatomen darstellen;
$R_4$ ein Halogenatom, ein Wasserstoffatom oder eine Alkoxygruppe, eine Gruppe

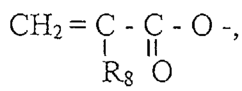

in welcher $R_8$ für H oder $CH_3$ steht, bezeichnet; und
$R_6$ die gleiche Bedeutung wie in Anspruch 3 hat.

5. Verbindungen nach Anspruch 4, dadurch gekennzeichnet, daß sie der folgenden Formel entsprechen:

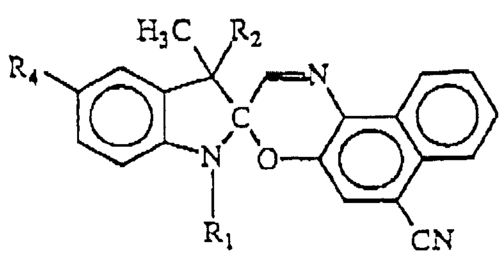

(I")

worin:

$R_1$ eine Alkylgruppe mit 1 bis 16 Kohlenstoffatomen oder eine Allylgruppe bezeichnet;
$R_2$ eine Alkylgruppe mit 1 bis 8 Kohlenstoffatomen bezeichnet;
$R_4$ ein Halogenatom, ein Wasserstoffatom, eine Alkoxygruppe mit 1 bis 6 Kohlenstoffatomen oder eine Gruppe

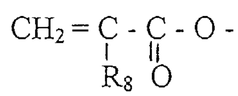

bezeichnet, wobei $R_8$ für H oder $CH_3$ steht.

**6.** Verbindungen nach Anspruch 5, dadurch gekennzeichnet, daß in der Formel (I"):

$R_1$ eine Methyl-, Hexadecyl- oder Allylgruppe bezeichnet;
$R_2$ eine Methyl- oder Ethylgruppe bezeichnet;
$R_4$ ein Wasserstoffatom, ein Chloratom oder eine Methoxygruppe oder $CH_2$=CH-COO- bezeichnet.

**7.** Verfahren zur Herstellung von Verbindungen nach einem der Ansprüche 1 bis 6, dadurch gekennzeichnet, daß es die Kondensation einer Fischerschen Base mit einem in Position 4 durch eine Gruppe $R_6$ substituierten Orthoaminophenol in einem Lösungsmittelmilieu in Gegenwart eines Oxidationsmittels umfaßt.

**8.** Verfahren zur Herstellung nach Anspruch 7, dadurch gekennzeichnet, daß es die Kondensation einer Fischerschen Base der Formel:

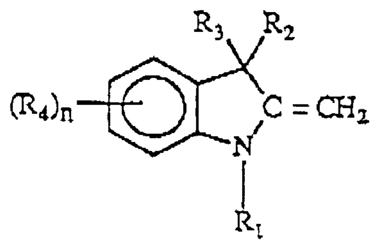

(II)

worin $R_1$, $R_2$, $R_3$, $R_4$ und n die in Anspruch 1 angegebenen Bedeutungen haben, mit einem anellierten Orthoaminophenol der Formel:

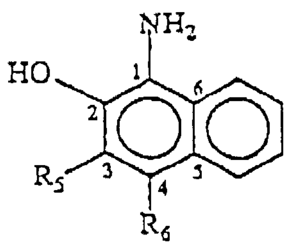

(III)

worin $R_5$ und $R_6$ die in Anspruch 3 angegebenen Bedeutungen haben, in einem Lösungsmittelmilieu in Gegenwart eines Oxidationsmittels umfaßt.

**9.** Verwendung einer Verbindung nach einem der Ansprüche 1 bis 6 als fotochrome Verbindung in der Augenoptik.

**10.** Zusammensetzung zur Aufbringung auf oder Einbringung in ein transparentes organisches Polymermaterial, dadurch gekennzeichnet, daß sie mindestens eine fotochrome Verbindung nach einem der Ansprüche 1 bis 6 in ausreichender Menge umfaßt, damit das Material bei Ultraviolettbestrahlung seine Farbe wechseln kann.

**11.** Zusammensetzung nach Anspruch 10, dadurch gekennzeichnet, daß die Zusammensetzung in flüssiger Form vorliegt und in gelöster oder dispergierter Form die fotochromen Verbindungen nach einem der Ansprüche 1 bis 6 in einem Medium auf Basis geeigneter Lösungsmittel umfaßt, um auf oder in ein transparentes Polymermaterial aufgebracht oder eingebracht zu werden.

**12.** Zusammensetzung zur Aufbringung auf oder Einbringung in ein transparentes organisches Polymermaterial, dadurch gekennzeichnet, daß sie aus einer farblosen oder transparenten Lösung auf Basis von Polymeren, Copolymeren oder einer Mischung von transparenten Polymeren in einem geeigneten organischen Lösungsmittel, enthaltend mindestens eine fotochrome Verbindung nach einem der Ansprüche 1 bis 6 in ausreichender Menge, damit das Material bei Ultraviolettbestrahlung seine Farbe wechseln kann besteht.

13. Transparenter Feststoff, welcher zur Herstellung von ophthalmischen Linsen geeignet ist, dadurch gekennzeichnet, daß er an der Oberfläche und/oder im Inneren mindestens eine fotochrome Verbindung nach einem der Ansprüche 1 bis 6 in ausreichender Menge umfaßt, damit das Material bei Ultraviolettbestrahlung seine Farbe wechseln kann.

14. Transparenter Feststoff nach Anspruch 13, dadurch gekennzeichnet, daß er 0,07 bis 20 Gew.% fotochrome Verbindungen enthält.

15. Transparente Zusammensetzung oder transparenter Feststoff nach einem der Ansprüche 10 bis 14, dadurch gekennzeichnet, daß die fotochrome Verbindung nach einem der Ansprüche 1 bis 6 gemeinsam mit anderen fotochromen Verbindungen, die unterschiedliche Färbungen ermöglichen, verwendet ist.

16. Transferlack, dadurch gekennzeichnet, daß er mindestens eine Verbindung nach einem der Ansprüche 1 bis 6 enthält.

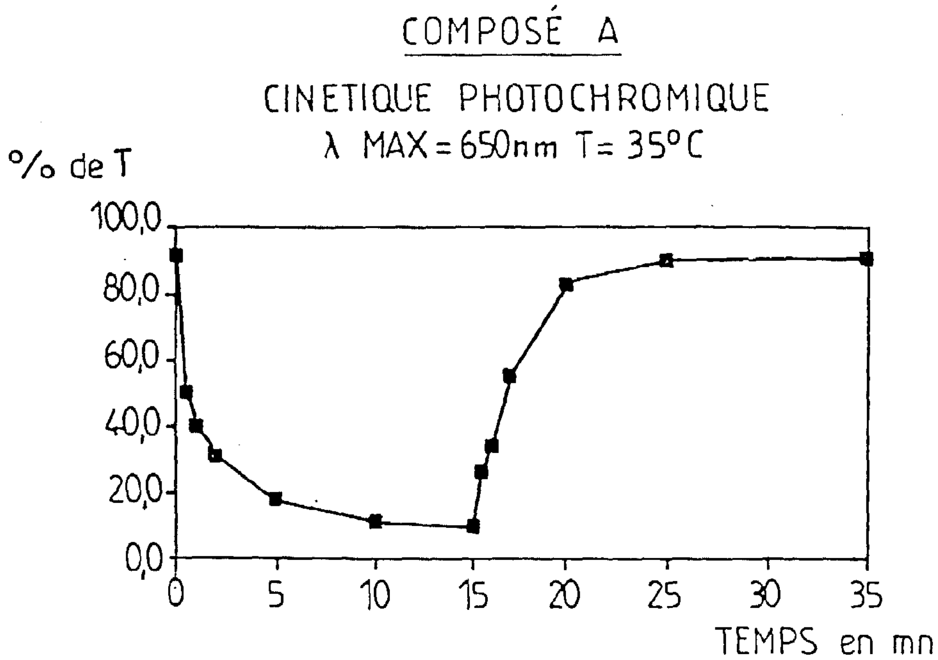
COMPOSÉ A
CINETIQUE PHOTOCHROMIQUE
λ MAX=650nm T= 35°C
% de T
100,0
80,0
60,0
40,0
20,0
0,0
0
5
10
15
20
25
30
35
TEMPS en mn

FIG.1

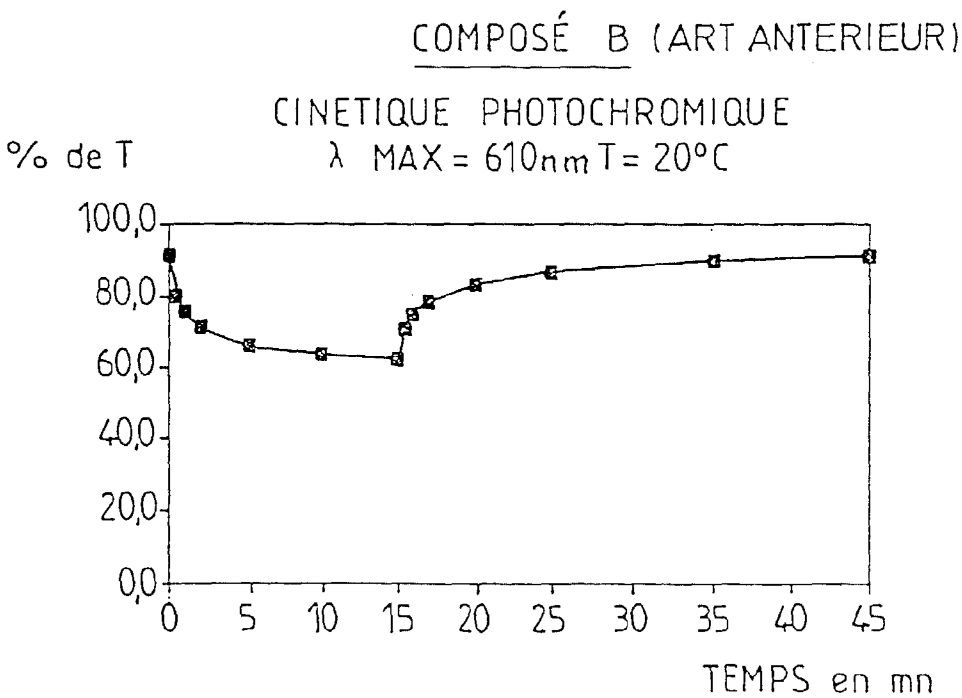
COMPOSÉ B (ART ANTERIEUR)
CINETIQUE PHOTOCHROMIQUE
λ MAX= 610nm T= 20°C
% de T
100,0
80,0
60,0
40,0
20,0
0,0
0
5
10
15
20
25
30
35
40
45
TEMPS en mn

FIG. 2

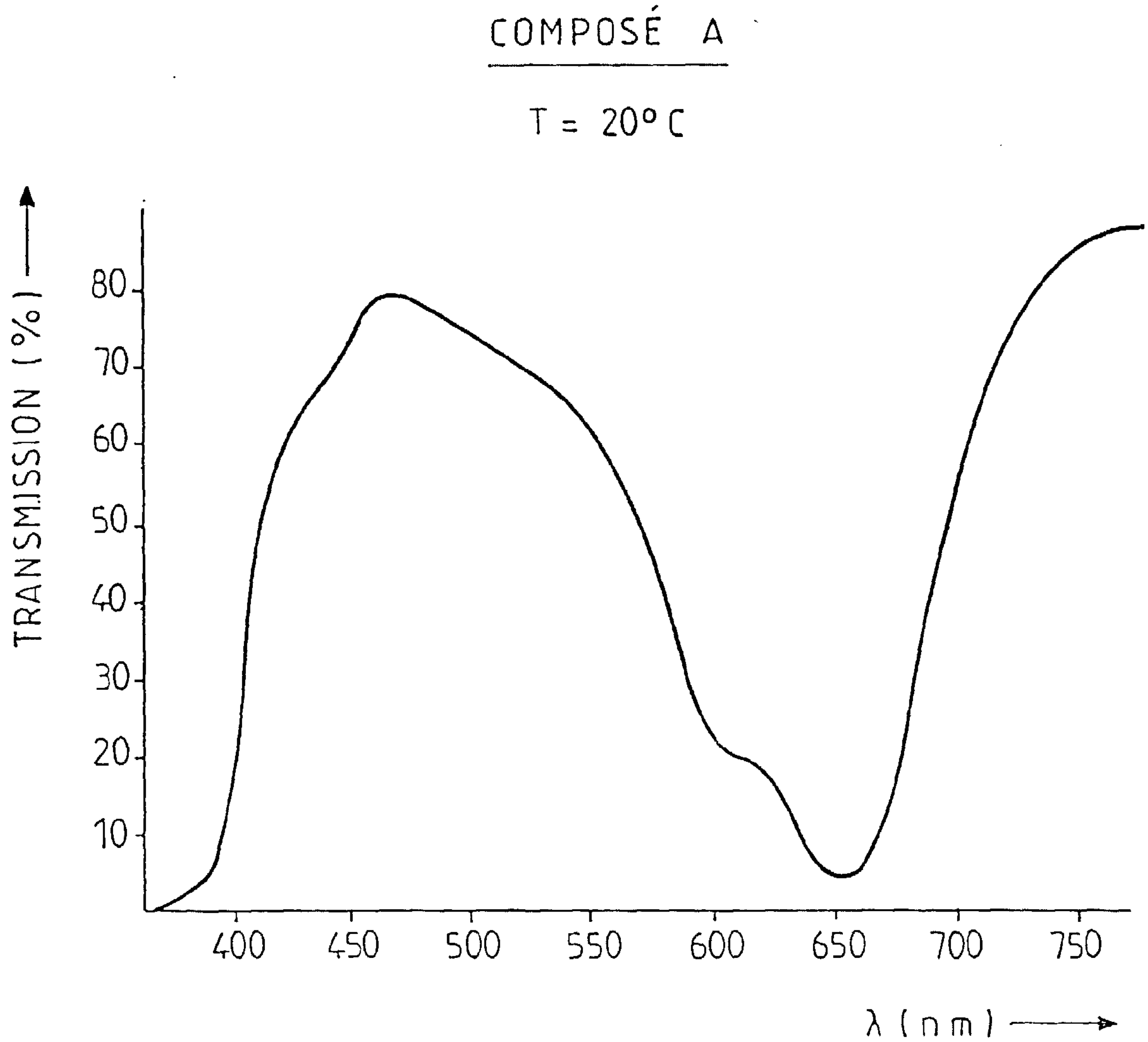
COMPOSÉ A
T = 20° C
TRANSMISSION (%)
80
70
60
50
40
30
20
10
400
450
500
550
600
650
700
750
λ (nm)

FIG.3

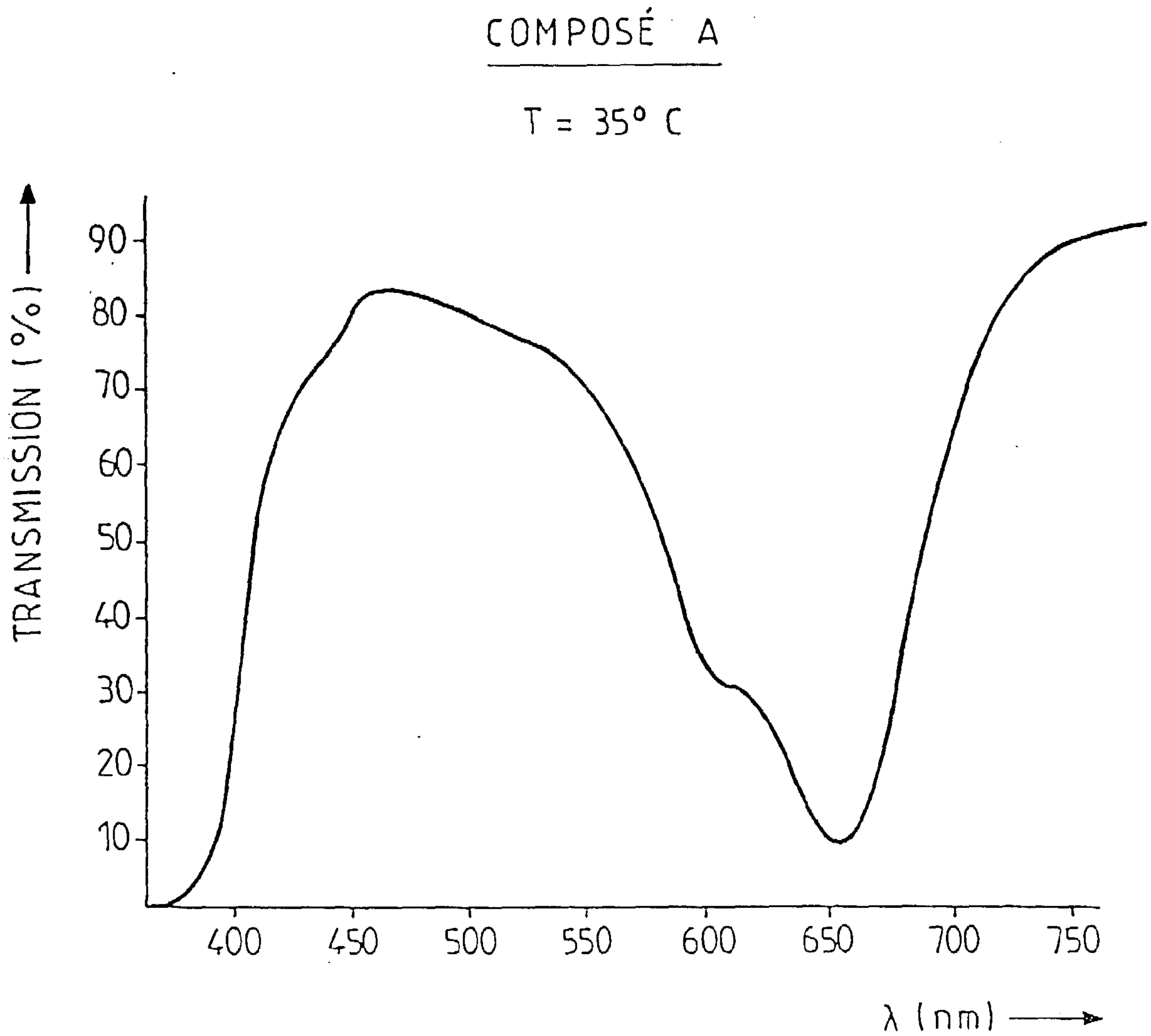
COMPOSÉ A
T = 35° C
TRANSMISSION (%)
90
80
70
60
50
40
30
20
10
400
450
500
550
600
650
700
750
λ (nm)

FIG.4

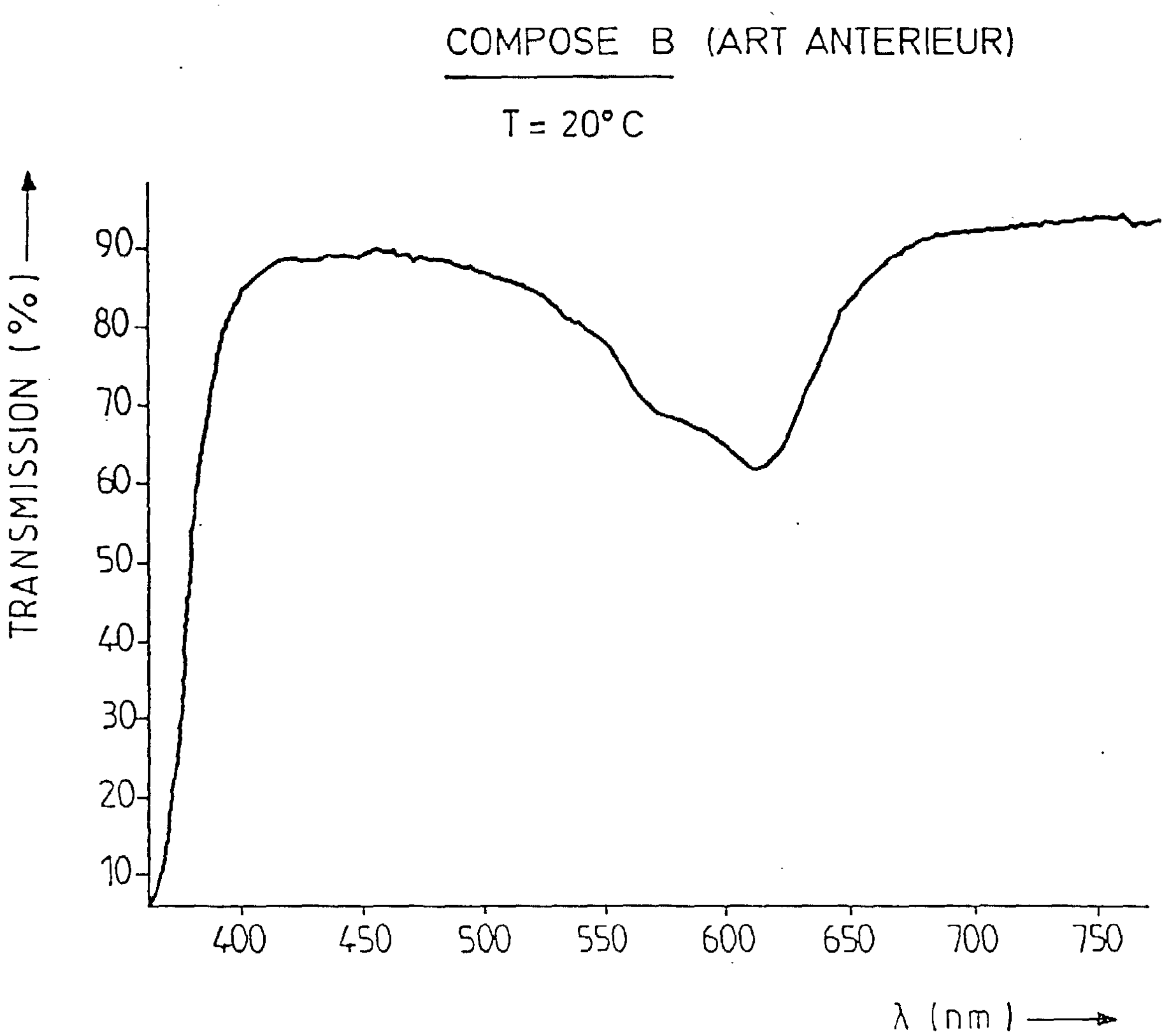
COMPOSE B (ART ANTERIEUR)
T = 20° C
TRANSMISSION (%)
90
80
70
60
50
40
30
20
10
400
450
500
550
600
650
700
750
λ (nm)

FIG. 5

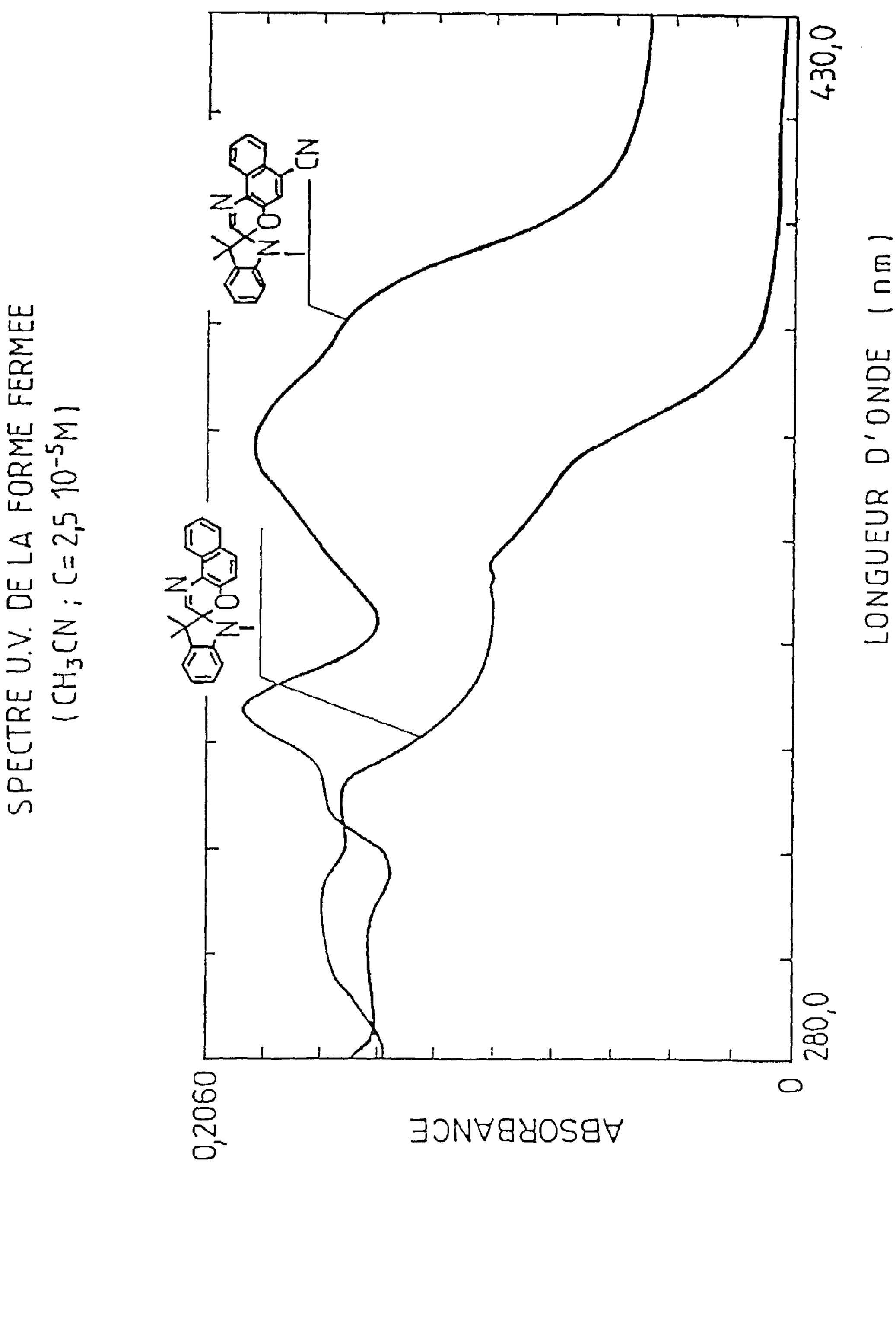
SPECTRE U.V. DE LA FORME FERMEE
( $CH_3CN$ ; C= 2,5 $10^{-5}M$ )
ABSORBANCE
0,2060
0
280,0
430,0
LONGUEUR D'ONDE (nm)

FIG. 6